# EUROPEAN PATENT SPECIFICATION

(11) **EP 2 934 571 B1**
(45) Date of publication and mention of the grant of the patent: **30.05.2018**
(21) Application number: 13818897.4
(22) Date of filing: 17.12.2013
(51) Int. Cl.: A61K 38/48, A61P 31/22

(54) **PROPHYLACTIC TREATMENT OF HERPES RECURRENCE**
PROPHYLAKTISCHE BEHANDLUNG VON HERPESREZIDIVEN
TRAITEMENT PROPHYLACTIQUE DE LA RÉCURRENCE DE L'HERPÈS

(30) Priority: 18.12.2012 US 201261738789 P
(43) Date of publication of application: 28.10.2015
(73) Proprietor: ALLERGAN, INC., Irvine, CA 92612 (US)
(72) Inventor: BRIN, Mitchell F., Newport Beach, California 92660 (US)
(74) Representative: Hoffmann Eitle
(86) International application number: PCT/US2013/075831
(87) International publication number: WO 2014/100019

(56) References cited:
- WO-A1-2009/158687
- US-A1- 2005 147 625
- US-A1- 2012 251 574
- DATABASE EMBASE [Online] ELSEVIER SCIENCE PUBLISHERS, AMSTERDAM, NL; January 2011 (2011-01), EMAD M R ET AL: "Botulinum toxin reduction pain in postherpetic neuralgia", XP002721181, Database accession no. EMB-2011051277 & EMAD M R ET AL: "Botulinum toxin reduction pain in postherpetic neuralgia", JOURNAL OF ISFAHAN MEDICAL SCHOOL 2011 ISFAHAN UNIVERSITY OF MEDICAL SCIENCES IRN, vol. 28, no. 114, January 2011 (2011-01), ISSN: 1027-7595

## Description

### CROSS-REFERENCE TO RELATED APPLICATIONS

This application claims the benefit of U.S. Provisional Application Serial No. 61/738,789, filed December 18, 2012.

### FIELD

The present invention relates to botulinum toxin for use in methods for prophylactic treatment of Herpes recurrence as defined in the claims.

### BACKGROUND

The Herpes virus is a pathogen which can affect humans as well as some higher order animals. The virus may cause periodic outbreaks of painful and contagious blisters at the site of infection. An initial infection is generally associated with an initial outbreak which may occur within 1-2 days of infection. Subsequent outbreaks may occur periodically and throughout the host's lifetime. Blisters can be located anywhere on the body but generally the site of infection is a mucus membrane, particularly the mouth, eyes and genitals. An infection may also occur where there is broken skin.

The Herpes virus is a lytic virus which lays dormant in the peripheral nervous system of its host establishing a latent infection for the lifetime of the host. The virus may lay dormant as a latent infection without the production of infective viral particles while maintaining the ability to reactivate, resume replication and cause recurrent symptoms. The latency process may be broken down into the following stages: viral replication at the peripheral site of infection, transport of viral particles to a latency site, establishment of latent infection, maintenance of the latent state for the life of the host and reactivation. Herpes simplex virus latent in the nervous system, Steiner, et al. Journal of NeuroVirology (1995) 1, 19-29.

Once infected, the virus travels up peripheral nerves to a nucleated neuron where it may lay dormant for years and subsequently followed by reactivation. The virus travels along the neuron by retrograde transport to the ganglion. In the case of oral mucosa herpes infection, the virus remains latent in the trigeminal ganglia. In the case of genital mucosa herpes infection, the virus remains latent in the sacral ganglia. Upon reactivation, the virus multiples in the nerve cell to produce new virus particles and travels along the axon of each neuron in the opposite direction to arrive at nerve endings in the skin at the original site of infection. Virus particles are released into the mucosa that was initially infected and vesicles containing infectious virus are formed on the mucosa and spreads to surrounding tissue and produce a lesion.

A reddened area gives rise to a macula which crusts to form a papula, generally referred to as a blister. The blister is a fluid filled lesion containing virus particles and is extremely infectious while moist. Initially, the lesion appears as a clear vesicle which contains infectious viral particles, the clear vesicle has at its base a red erythomatous lesion, collectively the initial lesion is referred to as the pustular. From the pustular, encrusted lesions and ulcers may develop. A dried blister then forms a scab, the scab may take up to 7 days to heal and may also contain contagious virus particles.

The virus may reactivate upon suppression of the host's immune system. Various factors may cause immune suppression including the presence of another organism, a disease or illness present in the host organism or increased stress levels of the host. In addition, an outbreak may be triggered by local stimuli, such as injury to tissues innervated by neurons harboring latent infection or by systemic conditions including exposure to the sun, fever, emotional stress or menstruation. Steiner, et al. Journal of NeuroVirology (1995) 1, 19-29.

Cell mediated responses are vital in controlling herpes infection, however these cell mediated responses, as well as inflammatory responses, may lead to some of the disease symptoms.

In addition to the physical effects the Herpes virus may have, there are also significant psychological effects of carrying the virus for an infected person. The virus may be transmitted even in the absence of an outbreak, thus to prevent transmission, it is important that any person carrying the virus inform others who may come in close contact with the carrier of the presence of the dormant virus. The presence of the Herpes virus may have a significant effect on any relationship the host may have with others who are in close contact with the host and may also prevent the creation of new intimate relationships for fear of contracting the virus.

Transmission of the virus may occur by directly contacting the infection site or by casual contact with items touched by a carrier. It is important that a carrier exercise extreme caution in preventing the transmission of the virus. To prevent casual transmission of the virus, the carrier is generally advised to maintain excellent personal hygiene, frequently wash their hands and avoid touching a blister during an outbreak. In addition, a carrier is advised to refrain from sharing food or utensils which may be contaminated with saliva containing the virus. Thus, reactivation of the virus may significantly affect a person's quality of life and may lead to negative personal consequences such as decreased self-esteem, distress, frustration, and/or avoidance of sexual intimacy.

Herpatic whitlow is a disease that affects health care workers which results in lesions on the fingers. Herpes gladiatorum is generally contracted by wrestlers and spreads from direct contact with a lesion. Infection may occur in the head and neck region.

Genital herpes may result in lesions on the glans or shaft of the penis in men and on the vulva, vagina, cervix and perianal region of women. The urethra may be infected in both sexes. Genital herpes may be accompanied by a variety of symptoms including fever, myalgia, and glandular inflammation of the groin area. Patients about experience a recurrence usually first experience a prodrome, which is an itching or burning sensation in the area that is about to erupt.

The Herpesvirales order is split into three families: Herpesviridae, Alloherpesviridae and Malacoherpesviridae. The Herpesvirdae family comprises at least 25 distinct virus types, at least 8 virus types affect humans, including Herpes simplex virus Type 1 (HSV-1), Herpes simplex virus Type 2 (HSV-2), Epstein Barr virus (EBV), Cytomegalovirus (CMV), Varicella Zoster virus (VZV), Human Herpes virus 6 (exanthum subitum or roseola infantum) and Human Herpes virus 8 (Kaposi's sarcoma-associated Herpes virus).

All herpes viruses are morphologically similar, with an overall size of 180 to 200 nm. Vioron structure of the herpes virus generally consists of a nucelocaspid portion which contains the DNA core of the viron, the linear double-stranded DNA genome is surrounded by an icosahedral capsid that is enclosed by glycoproteins containing lipid bilayer membrane, generally referred to as the envelope and derived predominantly from nuclear membrane. The envelope contains at least nine glycoproteins that protrude beyond it as spike-like structures. The space between capsid and the envelope is a protein-filled tegument, which contains viral proteins and enzymes that are required immediately for viral replication after infection. The viral genome is large, ranging from 125 to 240 kbp of DNA, which code for approximately 75 viral proteins. This large genome is necessary since Herpes viruses frequently infect nondividing cells and must therefore provide their own enzymes for DNA synthesis. Ray C.G., Ryan K.J. (2010). Chapter 14. Herpesviruses. In C.G. Ray, K.J. Ryan (Eds), Sherris Medical Microbiology, 5e.

There is no cure for a Herpes virus infection, however, drug treatments are available for minimizing the severity and duration of an outbreak. For example, antiviral medications may reduce the length and severity of an outbreak as well as reduce the recurrence of outbreaks. Drugs presently available include episodic therapy, which are administered at the onset of an outbreak, and suppressive therapy, which is taken daily to prevent an outbreak. Antiviral medications presently used include Acyclovir (Zovirax), Famciclovir (Famvir) and Valacyclovir (Valtrex). Topical medications include antiviral creams, such as Penciclovir (Denavir) and over the counter creams, such as docosanol (Abreva). However, side effects associated with consumption of these drugs may include difficulty breathing, nausea, vomiting, diarrhea, stomach pains, headache, unusual weakness and hives.

Other methods of treating a Herpes outbreak include the application of creams, lotions or balms to minimize the duration and severity of an outbreak or relieve pain associated with an outbreak.

There is a need for a prophylactic treatment of herpes recurrence. In particular, a method for preventing reactivation of the virus from the dormant state is desired to prevent recurrence of the virus and resulting symptoms. The present invention meets this need and provides for a prophylactic treatment for herpes recurrence as defined in the claims.

### SUMMARY OF THE INVENTION

The present invention meets the need for a new method for prophylactic treatment of Herpes recurrence by providing a safe and effective method to a patient in need thereof as defined in the claims.

In at least one embodiment, botulinum toxin for use in a method for prophylactic treatment of Herpes recurrence in a patient in need thereof is provided, the method comprising the step of locally administering said botulinum neurotoxin to the area at or near a ganglion wherein a Herpes virus lays dormant or at or near an infection site of the patient to thereby prophylactially treat the Herpes recurrence.

In certain embodiments, the method of prophylactic treatment of Herpes recurrence includes administration of a therapeutic amount of said botulinum neurotoxin by injection into the area at or near a peripheral nerve wherein the virus lays dormant.

In certain embodiments, the method of prophylactic treatment of Herpes recurrence includes local administration of a therapeutic amount of said botulinum neurotoxin by injection into the area at or near the infection site either alone or in combination with at least one injection into the base of the trigeminal nerve and/or facial muscles. In at least one further embodiment, the method of prophylactic treatment of Herpes recurrence includes the local administration of a therapeutic amount of a botulinum neurotoxin by injection into the area at or near the trigeminal nucleus caudalis, the ophthalmic branch, the maxiallry branch and/or the mandibular branch.

In certain embodiments, the method of prophylactic treatment of Herpes recurrence includes local administration of a therapeutic amount of said botulinum neurotoxin by injection into the area at or near the infection site either alone or in combination with at least one injection into the base of the facial nerve and/or facial muscles. In at least one further embodiment, the method of prophylactic treatment of Herpes recurrence includes the local administration of a therapeutic amount of a botulinum neurotoxin by injection into the area at or near the facial nerve and into the area at or near the temporal branch of the facial nerve, the zygomatic branch of the facial nerve, the buccal branch of the facial nerve, the marginal mandibular branch of the facial nerve or the cervical branch of the facial nerve.

In at least one further embodiment, the method of prophylactic treatment of Herpes recurrence includes the local administration of a therapeutic amount of a botulinum neurotoxin by injection into nerve or muscle tissue surrounding the mouth or eyes.

In at least one further embodiment, the method of prophylactic treatment of Herpes recurrence includes the local administration of a therapeutic amount of a botulinum neurotoxin by injection into the area near or at the genitofemoral nerve, pudendal nerve, external spermatic nerve, sacral nerve, sciatic nerve, dorsal nerve, perineal nerve or posterior scrotal nerve.

A preferable botulinum neurotoxin for use in the methods herein described is a botulinum neurotoxin, which can be selected from the group consisting of botulinum neurotoxin types A, B, C, D, E, F and G, and is preferably botulinum neurotoxin type A. Various ranges/amount of botulinum neurotoxin can be therapeutically administered in accordance with the teachings of the present disclosure, for example, botulinum neurotoxin can be administered in an amount of from about 1 unit to about 20,000 units, dependent, of course, on the potency of the botulinum neurotoxin type utilized and its method of administration (e.g. an amount of botulinum neurotoxin contained in a slow-release implant or pulsatile implant can be many times greater than an amount of botulinum neurotoxin that is administered directly and at once, rather than slowly released from an implant). Exemplary useful amounts for a botulinum neurotoxin type A or type B, can be from about 1 unit to about 2500 units, or from about 10 to about 15,000 units, or from about 25 to about 1000 units respectively, or an amount or range therebetween.

Botulinum neurotoxin may also be administered in an amount less than that which is needed to paralyze a muscle.

In an additional embodiment, prophylactic treatment of Herpes recurrence could be by administration of a targeted exocytosis modulator ("TEM") according the the injection paradigms disclosed herein for botulinum neurotoxins.

### DESCRIPTION

The genus Clostridium encompasses over one hundred and twenty seven species, grouped according to their morphology and functions. The anaerobic, gram positive bacterium Clostridium botulinum produces a potent polypeptide neurotoxin, botulinum neurotoxin, which causes a neuroparalytic illness in humans and animals referred to as botulism. The spores of Clostridium botulinum are found in soil and can grow in improperly sterilized and sealed food containers of home based canneries, which are the cause of many of the cases of botulism. The effects of botulism typically appear 18 to 36 hours after eating the foodstuffs infected with a Clostridium botulinum culture or spores. The botulinum neurotoxin can apparently pass unattenuated through the lining of the gut and attack peripheral motor neurons. Symptoms of botulinum neurotoxin intoxication can progress from difficulty walking, swallowing, and speaking to paralysis of the respiratory muscles and death.

Botulinum neurotoxin type A is the most lethal natural biological agent known to man. About 50 picograms of a commercially available botulinum neurotoxin type A (purified neurotoxin complex) is a LD₅₀ in mice *(i.e.* 1 unit). One unit of BOTOX® contains about 50 picograms (about 56 attomoles) of botulinum neurotoxin type A complex. Interestingly, on a molar basis, botulinum neurotoxin type A is about 1.8 billion times more lethal than diphtheria, about 600 million times more lethal than sodium cyanide, about 30 million times more lethal than cobra toxin and about 12 million times more lethal than cholera. Singh, Critical Aspects of Bacterial Protein Toxins, pages 63-84 (chapter 4) of Natural Toxins II, edited by B. R. Singh et al., Plenum Press, New York (1976) (where the stated LD₅₀ of botulinum neurotoxin type A of 0.3 ng equals 1 U is corrected for the fact that about 0.05 ng of BOTOX® equals 1 unit). One unit (U) of botulinum neurotoxin was initially defined as the LD₅₀ upon intraperitoneal injection into female Swiss Webster mice weighing 18 to 20 grams each. Now, a cell based potency assay has been approved in many jurisdictions which decreased the amount of animal testing. See US Patent 8,198,034. One example of a botulinum nerurotoxin therapeutic is Botox®, available from Allergan, Inc., of Irvine, Calififornia.

Seven generally immunologically distinct botulinum neurotoxins have been characterized, these being respectively botulinum neurotoxin serotypes A, B, C₁, D, E, F and G each of which is distinguished by neutralization with type-specific antibodies. The different serotypes of botulinum neurotoxin vary in the animal species that they affect and in the severity and duration of the paralysis they evoke. For example, it has been determined that botulinum neurotoxin type A is 500 times more potent, as measured by the rate of paralysis produced in the rat, than is botulinum neurotoxin type B. Additionally, botulinum neurotoxin type B has been determined to be non-toxic in primates at a dose of 480 U/kg which is about 12 times the primate LD₅₀ for botulinum neurotoxin type A. Moyer E. et al., Botulinum Toxin Type B: Experimental and Clinical Experience, being chapter 6, pages 71-85 of "Therapy With Botulinum Toxin", edited by Jankovic, J. et al. (1994), Marcel Dekker, Inc. Botulinum toxin apparently binds with high affinity to cholinergic motor neurons, is translocated into the neuron and blocks the release of acetylcholine. Additional uptake can take place through low affinity receptors, as well as by phagocytosis and pinocytosis.

Regardless of serotype, the molecular mechanism of toxin intoxication appears to be similar and to involve at least three steps or stages. In the first step of the process, the toxin binds to the presynaptic membrane of the target neuron through a specific interaction between the heavy chain, H chain, and a cell surface receptor; the receptor is thought to be different for each type of botulinum neurotoxin and for tetanus toxin. The carboxyl end segment of the H chain, H_{C}, appears to be important for targeting of the toxin to the cell surface.

In the second step, the toxin crosses the plasma membrane of the poisoned cell. The toxin is first engulfed by the cell through receptor-mediated endocytosis, and an endosome containing the toxin is formed. The toxin then escapes the endosome into the cytoplasm of the cell. This step is thought to be mediated by the amino end segment of the H chain, H_{N}, which triggers a conformational change of the toxin in response to a pH of about 5.5 or lower. Endosomes are known to possess a proton pump which decreases intra-endosomal pH. The conformational shift exposes hydrophobic residues in the toxin, which permits the toxin to embed itself in the endosomal membrane. The toxin (or at a minimum the light chain) then translocates through the endosomal membrane into the cytoplasm.

The last step of the mechanism of botulinum neurotoxin activity appears to involve reduction of the disulfide bond joining the heavy chain, H chain, and the light chain, L chain. The entire toxic activity of botulinum and tetanus toxins is contained in the L chain of the holotoxin; the L chain is a zinc (Zn++) endopeptidase which selectively cleaves proteins essential for recognition and docking of neurotransmitter-containing vesicles with the cytoplasmic surface of the plasma membrane, and fusion of the vesicles with the plasma membrane. Tetanus neurotoxin, botulinum neurotoxin types B, D, F, and G cause degradation of synaptobrevin (also called vesicle-associated membrane protein (VAMP)), a synaptosomal membrane protein. Most of the VAMP present at the cytoplasmic surface of the synaptic vesicle is removed as a result of any one of these cleavage events. Botulinum neurotoxin serotypes A and E cleave SNAP-25. Botulinum neurotoxin serotype C₁ was originally thought to cleave syntaxin, but was found to cleave syntaxin and SNAP-25. Each of the botulinum neurotoxins specifically cleaves a different bond, except botulinum neurotoxin type B (and tetanus toxin) which cleave the same bond. Each of these cleavages block the process of vesicle-membrane docking, thereby preventing exocytosis of vesicle content.

Although all the botulinum neurotoxins serotypes apparently inhibit release of the neurotransmitter acetylcholine at the neuromuscular junction, they do so by affecting different neurosecretory proteins and/or cleaving these proteins at different sites. For example, botulinum types A and E both cleave the 25 kiloDalton (kDa) synaptosomal associated protein (SNAP-25), but they target different amino acid sequences within this protein. Botulinum neurotoxin types B, D, F and G act on vesicle-associated protein (VAMP, also called synaptobrevin), with each serotype cleaving the protein at a different site. Finally, botulinum neurotoxin type C₁ has been shown to cleave both syntaxin and SNAP-25. These differences in mechanism of action may affect the relative potency and/or duration of action of the various botulinum neurotoxin serotypes.

The molecular weight of the botulinum neurotoxin protein molecule, for all seven of the known botulinum neurotoxin serotypes, is about 150kDa. Interestingly, the botulinum neurotoxins are released by Clostridial bacterium as complexes comprising the 150kDa botulinum neurotoxin protein molecule along with associated non-toxin proteins. Thus, the botulinum neurotoxin type A complex can be produced by Clostridial bacterium as 900kDa, 500kDa and 300kDa forms. Botulinum neurotoxin types B and C1 are apparently produced as only a 700kD or 500kD complex. Botulinum neurotoxin type D is produced as both 300kD and 500kD complexes. Finally, botulinum neurotoxin types E and F are produced as only approximately 300kDa complexes. The complexes *(i.e.* molecular weight greater than about 150kDa) are believed to contain a non-toxin hemaglutinin protein and a non-toxin and non-toxic nonhemaglutinin protein. These two non-toxin proteins (which along with the botulinum neurotoxin molecule comprise the relevant neurotoxin complex) may act to provide stability against denaturation to the botulinum neurotoxin molecule and protection against digestive acids when toxin is ingested. Additionally, it is possible that the larger (greater than about 150kDa molecular weight) botulinum neurotoxin complexes may result in a slower rate of diffusion of the botulinum neurotoxin away from a site of intramuscular injection of a botulinum neurotoxin complex.

In vitro studies have indicated that botulinum neurotoxin inhibits potassium cation induced release of both acetylcholine and norepinephrine from primary cell cultures of brainstem tissue. Additionally, it has been reported that botulinum neurotoxin inhibits the evoked release of both glycine and glutamate in primary cultures of spinal cord neurons and that in brain synaptosome preparations botulinum neurotoxin inhibits the release of each of the neurotransmitters acetylcholine, dopamine, norepinephrine (Habermann E., et al., Tetanus Toxin and Botulinum A and C Neurotoxins Inhibit Noradrenaline Release From Cultured Mouse Brain, J Neurochem 51(2); 522-527:1988) CGRP, substance P and glutamate (Sanchez-Prieto, J., et al., Botulinum Toxin A Blocks Glutamate Exocytosis From Guinea Pig Cerebral Cortical Synaptosomes, Eur J. Biochem 165; 675-681:1897. Thus, when adequate concentrations are used, stimulus-evoked release of most neurotransmitters is blocked by botulinum neurotoxin. See *e.g.* Pearce, L. B., Pharmacologic Characterization of Botulinum Toxin For Basic Science and Medicine, Toxicon 35(9); 1373-1412 at 1393; Bigalke H., et al., Botulinum A Neurotoxin Inhibits Non-Cholinergic Synaptic Transmission in Mouse Spinal Cord Neurons in Culture, Brain Research 360; 318-324:1985; Habermann E., Inhibition by Tetanus and Botulinum A Toxin of the release of [3H]Noradrenaline and [3H]GABA From Rat Brain Homogenate, Experientia 44; 224-226:1988, Bigalke H., et al., Tetanus Toxin and Botulinum A Toxin Inhibit Release and Uptake of Various Transmitters, as Studied with Particulate Preparations From Rat Brain and Spinal Cord, Naunyn-Schmiedeberg's Arch Pharmacol 316; 244-251:1981, and; Jankovic J. et al., Therapy With Botulinum Toxin, Marcel Dekker, Inc., (1994), page 5.

Botulinum neurotoxin type A can be obtained by establishing and growing cultures of Clostridium botulinum in a fermenter and then harvesting and purifying the fermented mixture in accordance with known procedures. All the botulinum neurotoxin serotypes are initially synthesized as inactive single chain proteins which must be cleaved or nicked by proteases to become neuroactive. The bacterial strains that make botulinum neurotoxin serotypes A and G possess endogenous proteases and serotypes A and G can therefore be recovered from bacterial cultures in predominantly their active form. In contrast, botulinum neurotoxin serotypes C₁, D and E are synthesized by nonproteolytic strains and are therefore typically unactivated when recovered from culture. Serotypes B and F are produced by both proteolytic and nonproteolytic strains and therefore can be recovered in either the active or inactive form. However, even the proteolytic strains that produce, for example, the botulinum neurotoxin type B serotype only cleave a portion of the toxin produced. The exact proportion of nicked to unnicked molecules depends on the length of incubation and the temperature of the culture. Therefore, a certain percentage of any preparation of, for example, the botulinum neurotoxin type B toxin is likely to be inactive, possibly accounting for the known significantly lower potency of botulinum neurotoxin type B as compared to botulinum neurotoxin type A. The presence of inactive botulinum neurotoxin molecules in a clinical preparation will contribute to the overall protein load of the preparation, which has been linked to increased antigenicity, without contributing to its clinical efficacy. Additionally, it is known that botulinum neurotoxin type B has, upon intramuscular injection, a shorter duration of activity and is also less potent than botulinum neurotoxin type A at the same dose level.

High quality crystalline botulinum neurotoxin type A can be produced from the Hall A strain of Clostridium botulinum with characteristics of 3 times 10⁷ U/mg, an A₂₆₀/A₂₇₈ of less than 0.60 and a distinct pattern of banding on gel electrophoresis. The known Schantz process can be used to obtain crystalline botulinum neurotoxin type A, as set forth in Schantz, E. J., et al, Properties and use of Botulinum toxin and Other Microbial Neurotoxins in Medicine, Microbiol Rev. 56; 80-99:1992. Generally, the botulinum neurotoxin type A complex can be isolated and purified from an anaerobic fermentation by cultivating Clostridium botulinum type A in a suitable medium. The known process can also be used, upon separation out of the non-toxin proteins, to obtain pure botulinum neurotoxins, such as for example: purified botulinum neurotoxin type A with an approximately 150kDa molecular weight with a specific potency of 1-2 times 10⁸ LD₅₀ U/mg or greater; purified botulinum neurotoxin type B with an approximately 156kDa molecular weight with a specific potency of 1-2 times 10⁸ LD₅₀ U/mg or greater, and; purified botulinum neurotoxin type F with an approximately 155kDa molecular weight with a specific potency of 1-2 times 10⁷ LD₅₀ U/mg or greater.

A commercially available botulinum neurotoxin containing pharmaceutical composition is sold under the trademark BOTOX® (available from Allergan, Inc., of Irvine, Calif.). BOTOX® consists of a purified botulinum neurotoxin type A complex, albumin and sodium chloride packaged in sterile, vacuum-dried form. The botulinum neurotoxin type A is made from a culture of the Hall strain of Clostridium botulinum grown in a medium containing N-Z amine and yeast extract. The botulinum neurotoxin type A complex is purified from the culture solution by a series of acid precipitations to a crystalline complex consisting of the active high molecular weight toxin protein and an associated hemagglutinin protein. The crystalline complex is redissolved in a solution containing saline and albumin and sterile filtered (0.2 microns) prior to vacuum-drying. The vacuum-dried product is stored in a freezer at or below -5C. BOTOX® can be reconstituted with sterile, non-preserved saline prior to intramuscular injection. Each vial of BOTOX® contains about 50 units (U), 100 U or 200 U of Clostridium botulinum neurotoxin type A purified neurotoxin complex.

To reconstitute vacuum-dried BOTOX®, sterile normal saline without a preservative; (0.9% Sodium Chloride Injection) is used by drawing up the proper amount of diluent in the appropriate size syringe. Because BOTOX® may be denatured by bubbling or similar violent agitation, the diluent is gently injected into the vial. For sterility reasons BOTOX® is usually administered within four hours after the vial is removed from the freezer and reconstituted. During these four hours, reconstituted BOTOX® can be stored in a refrigerator at about 2C to about 8C. Reconstituted, refrigerated BOTOX® has been reported to retain its potency for at least about two weeks. Neurology, 48:249-53:1997.

It has been reported that botulinum neurotoxin type A has been used in clinical settings as follows: use of BOTOX® for intramuscular injection (multiple muscles) to treat cervical dystonia; use of BOTOX® for intramuscular injection (e.g. procerus muscle and/or corrugator supercihii muscles) to treat glabellar lines (brow furrows); use of BOTOX® to treat constipation by intrasphincter injection of the puborectalis muscle; use of BOTOX® for intramuscular injection to treat blepharospasm by injecting the lateral pre-tarsal orbicularis oculi muscle of the upper lid and the lateral pre-tarsal orbicularis oculi of the lower lid; use of BOTOX® for intramuscular injection (e.g. extraocular muscles) to treat strabismus, the amount injected varying based upon both the size of the muscle to be injected and the extent of muscle paralysis desired *(i.e.* amount of diopter correction desired); use of BOTOX® to treat upper limb spasticity following stroke by intramuscular injections, for example by injection into one or more of five different upper limb flexor muscles, as follows: (a) flexor digitorum profundus (e.g. 7.5U to 30U), (b) flexor digitorum sublimes (e.g. 7.5U to 30U), (c) flexor carpi ulnaris (e.g. 10U to 40U), (d) flexor carpi radialis (e.g. 15U to 60U), and (e) biceps brachii (e.g. 50U to 200U); use of BOTOX® to treat migraine, for example by pericranial injection symmetrically into glabellar, frontalis and temporalis muscles, or for example by injection into frontalis, corrugator, procerus, occipitalis, temporalis, trapezius and cervical paraspinal muscle groups, as a prophylactic treatment of migraine compared to vehicle as measured by decreased measures of migraine frequency, maximal severity, associated vomiting and/or acute medication use over a three month period following injection; and use of BOTOX® to treat detrusor overactivity associated with a neurological condition, for example by injection of 200U into the detrusor muscle.

Clinical effects of peripheral intramuscular botulinum neurotoxin type A are usually seen within one week of injection. The typical duration of symptomatic relief from a single intramuscular injection of botulinum neurotoxin type A averages about three months, although significantly longer periods of therapeutic activity have been reported.

The success of botulinum neurotoxin type A to treat a variety of clinical conditions has led to interest in other botulinum neurotoxin serotypes. Two other commercially available botulinum type A preparations for use in humans in the United States include DYSPORT® available from Beaufour Ipsen, Porton Down, England, and Xeomin® from Merz Pharmaceuticals. A botulinum neurotoxin type B preparation (MYOBLOC®) is available from Elan Pharmaceuticals of San Francisco, Calif.

A Clostridial toxin treatment inhibits neurotransmitter release by disrupting the exocytotic process used to secret the neurotransmitter into the synaptic cleft. There is a great desire by the pharmaceutical industry to expand the use of Clostridial toxin therapies beyond its current myo-relaxant applications to treat sensory nerve-based ailment, such as, e.g., various kinds of chronic pain, neurogenic inflammation and urogentital disorders, as well as other disorders, such as, e.g., pancreatitis. One approach to expand the use of Clostridial toxin-based therapies involves modifying a Clostridial toxin so that the modified toxin has an altered cell targeting capability. This re-targeted capability is achieved by replacing a naturally-occurring targeting domain of a Clostridial toxin with a targeting domain having a binding activity for a non-Clostridial toxin receptor. Called Targeted Vesicular Exocytosis Modulating Proteins (TEMs), these retargeted molecules bind to a non-Clostridial toxin receptor, internalize into the cytoplasm, translocate the enzymatic domain into the cytoplasm, and exert a proteolytic effect on a component of the SNARE complex of the target cell.

However, an important difference between TEMs, such as, *e.g.,* TEMs disclosed herein, and native Clostridial toxins is that since TEMs do not target motor neurons, the lethality associated with over-dosing an individual with a TEM is greatly minimized, if not avoided altogether. For example, a TEM comprising an opioid targeting domain can be administered at 10,000 times the therapeutically effective dose before evidence of lethality is observed, and this lethality is due to the passive diffusion of the molecule and not via the intoxication process. Thus, for all practical purposes TEMs are non-lethal molecules.

Aspects of the present specification disclose, in part, a Targeted Vesicular Exocytosis Modulator Protein. As used herein, the term Targeted Vesicular Exocytosis Modulator Protein" is synonymous with "TEM" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

As used herein, the term "Clostridial toxin enzymatic domain" refers to a Clostridial toxin polypeptide located in the light chain of a Clostridial toxin that executes the enzymatic target modification step of the intoxication process. A Clostridial toxin enzymatic domain includes a metalloprotease region containing a zinc-dependent endopeptidase activity which specifically targets core components of the neurotransmitter release apparatus. Thus, a Clostridial toxin enzymatic domain specifically targets and proteolytically cleavages of a Clostridial toxin substrate, such as, e.g., SNARE proteins like a SNAP-25 substrate, a VAMP substrate and a Syntaxin substrate.

A Clostridial toxin enzymatic domain includes, without limitation, naturally occurring Clostridial toxin enzymatic domain variants, such as, e.g., Clostridial toxin enzymatic domain isoforms and Clostridial toxin enzymatic domain subtypes; non-naturally occurring Clostridial toxin enzymatic domain variants, such as, e.g., conservative Clostridial toxin enzymatic domain variants, non-conservative Clostridial toxin enzymatic domain variants, Clostridial toxin enzymatic domain chimeras, active Clostridial toxin enzymatic domain fragments thereof, or any combination thereof. Non-limiting examples of a Clostridial toxin enzymatic domain include, *e.g.,* a BoNT/A enzymatic domain, a BoNT/B enzymatic domain, a BoNT/C1 enzymatic domain, a BoNT/D enzymatic domain, a BoNT/E enzymatic domain, a BoNT/F enzymatic domain, a BoNT/G enzymatic domain, a TeNT enzymatic domain, a BaNT enzymatic domain, and a BuNT enzymatic domain.

As used herein, the term "Clostridial toxin translocation domain" refers to a Clostridial toxin polypeptide located within the amino-terminal half of the heavy chain of a Clostridial toxin that executes the translocation step of the intoxication process. The translocation step appears to involve an allosteric conformational change of the translocation domain caused by a decrease in pH within the intracellular vesicle. This conformational change results in the formation of a pore in the vesicular membrane that permits the movement of the light chain from within the vesicle into the cytoplasm. Thus, a Clostridial toxin translocation domain facilitates the movement of a Clostridial toxin light chain across a membrane of an intracellular vesicle into the cytoplasm of a cell.

A Clostridial toxin translocation domain includes, without limitation, naturally occurring Clostridial toxin translocation domain variants, such as, e.g., Clostridial toxin translocation domain isoforms and Clostridial toxin translocation domain subtypes; non-naturally occurring Clostridial toxin translocation domain variants, such as, e.g., conservative Clostridial toxin translocation domain variants, non-conservative Clostridial toxin translocation domain variants, Clostridial toxin translocation domain chimerics, active Clostridial toxin translocation domain fragments thereof, or any combination thereof. Non-limiting examples of a Clostridial toxin translocation domain include, *e.g.,* a BoNT/A translocation domain, a BoNT/B translocation domain, a BoNT/C1 translocation domain, a BoNT/D translocation domain, a BoNT/E translocation domain, a BoNT/F translocation domain, a BoNT/G translocation domain, a TeNT translocation domain, a BaNT translocation domain, and a BuNT translocation domain.

As used herein, the term "targeting domain" is synonymous with "binding domain" or "targeting moiety" and refers to a polypeptide that executes the receptor binding and/or complex internalization steps of the intoxication process, with the proviso that the binding domain is not a Clostridial toxin binding domain found within the carboxyl-terminal half of the heavy chain of a Clostridial toxin. A targeting domain includes a receptor binding region that confers the binding activity and/or specificity of the targeting domain for its cognate receptor. As used herein, the term "cognate receptor" refers to a receptor for which the targeting domain preferentially interacts with under physiological conditions, or under in vitro conditions substantially approximating physiological conditions. As used herein, the term "preferentially interacts" is synonymous with "preferentially binding" and refers to an interaction that is statistically significantly greater in degree relative to a control. With reference to a targeting domain disclosed herein, a targeting domain binds to its cognate receptor to a statistically significantly greater degree relative to a non-cognate receptor. Said another way, there is a discriminatory binding of the targeting domain to its cognate receptor relative to a non-cognate receptor. Thus, a targeting domain directs binding to a TEM-specific receptor located on the plasma membrane surface of a target cell.

In an embodiment, a targeting domain disclosed herein has an association rate constant that confers preferential binding to its cognate receptor. In aspects of this embodiment, a targeting domain disclosed herein binds to its cognate receptor with an association rate constant of, *e.g.*, less than 1 x 10⁵ M⁻¹ s⁻¹, less than 1 x 10⁶ M⁻¹ s⁻¹, less than 1 x 10⁷ M⁻¹ s⁻¹, or less than 1 x 10⁸ M⁻¹ s⁻¹. In other aspects of this embodiment, a targeting domain disclosed herein binds to its cognate receptor with an association rate constant of, *e.g.,* more than 1 x 10⁵ M⁻¹ s⁻¹, more than 1 x 10⁶ M⁻¹ s⁻¹, more than 1 x 10⁷ M⁻¹ s⁻¹, or more than 1 x 10⁸ M⁻¹ s⁻¹. In yet other aspects of this embodiment, a targeting domain disclosed herein binds to its cognate receptor with an association rate constant between 1 x 10⁵ M⁻¹ s⁻¹ to 1 x 10⁸ M⁻¹ s⁻¹, 1 x 10⁶ M⁻¹ s⁻¹ to 1 x 10⁸ M⁻¹ s⁻¹, 1 x 10⁵ M⁻¹ s⁻¹ to 1 x 10⁷ M⁻¹ s⁻¹, or 1 x 10⁶ M⁻¹ s⁻¹ to 1 x 10⁷ M⁻¹ s⁻¹.

In another embodiment, a targeting domain disclosed herein has an association rate constant that is greater for its cognate target receptor relative to a non-cognate receptor. In other aspects of this embodiment, a targeting domain disclosed herein has an association rate constant that is greater for its cognate target receptor relative to a non-cognate receptor by, at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has an association rate constant that is greater for its cognate target receptor relative to a non-cognate receptor by, e.g., about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In yet another embodiment, a targeting domain disclosed herein has a disassociation rate constant that confers preferential binding to its cognate receptor. In other aspects of this embodiment, a targeting domain disclosed herein binds to its cognate receptor with a disassociation rate constant of less than 1 x 10⁻³ s⁻¹, less than 1 x 10⁻⁴ s⁻¹, or less than 1 x 10⁻⁵ s⁻¹. In yet other aspects of this embodiment, a targeting domain disclosed herein binds to its cognate receptor with a disassociation rate constant of, *e.g.,* less than 1.0 x 10⁻⁴ s⁻¹, less than 2.0 x 10⁻⁴ s⁻¹, less than 3.0 x 10⁻⁴ s⁻¹, less than 4.0 x 10⁻⁴ s⁻¹, less than 5.0 x 10⁻⁴ s⁻¹, less than 6.0 x 10⁻⁴ s⁻¹, less than 7.0 x 10⁻⁴ s⁻¹, less than 8.0 x 10⁻⁴ s⁻¹, or less than 9.0 x 10⁻⁴ s⁻¹. In still other aspects of this embodiment, a targeting domain disclosed herein binds to its cognate receptor with a disassociation rate constant of, *e.g.,* more than 1 x 10⁻³ s⁻¹, more than 1 x 10⁻⁴ s⁻¹, or more than 1 x 10⁻⁵ s⁻¹. In other aspects of this embodiment, a targeting domain disclosed herein binds to its cognate receptor with a disassociation rate constant of, *e.g.,* more than 1.0 x 10⁻⁴ s⁻¹, more than 2.0 x 10⁻⁴ s⁻¹, more than 3.0 x 10⁻⁴ s⁻¹, more than 4.0 x 10⁻⁴ s⁻¹, more than 5.0 x 10⁻⁴ s⁻¹, more than 6.0 x 10⁻⁴ s⁻¹, more than 7.0 x 10⁻⁴ s⁻¹, more than 8.0 x 10⁻⁴ s⁻¹, or more than 9.0 x 10⁻⁴ s⁻¹.

In still another embodiment, a targeting domain disclosed herein has a disassociation rate constant that is less for its cognate target receptor relative to a non-cognate receptor. In other aspects of this embodiment, a targeting domain disclosed herein has a disassociation rate constant that is less for its cognate target receptor relative to a non-cognate receptor by, e.g., at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has a disassociation rate constant that is less for its cognate target receptor relative to a non-cognate receptor by, e.g., about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In another embodiment, a targeting domain disclosed herein has an equilibrium disassociation constant that confers preferential binding to its cognate receptor. In other aspects of this embodiment, a targeting domain disclosed herein binds to its cognate receptor with an equilibrium disassociation constant of, *e.g.,* less than 0.500 nM. In yet other aspects of this embodiment, a targeting domain disclosed herein binds to its cognate receptor with an equilibrium disassociation constant of, *e.g.,* less than 0.500 nM, less than 0.450 nM, less than 0.400 nM, less than 0.350 nM, less than 0.300 nM, less than 0.250 nM, less than 0.200 nM, less than 0.150 nM, less than 0.100 nM, or less than 0.050 nM. In other aspects of this embodiment, a targeting domain disclosed herein binds to its cognate receptor with an equilibrium disassociation constant of, *e.g.,* more than 0.500 nM, more than 0.450 nM, more than 0.400 nM, more than 0.350 nM, more than 0.300 nM, more than 0.250 nM, more than 0.200 nM, more than 0.150 nM, more than 0.100 nM, or more than 0.050 nM.

In yet another embodiment, a targeting domain disclosed herein has an equilibrium disassociation constant that is greater for its cognate target receptor relative to a non-cognate receptor. In other aspects of this embodiment, a targeting domain disclosed herein has an equilibrium disassociation constant that is greater for its cognate target receptor relative to a non-cognate receptor by, *e.g.,* at least one-fold, at least two-fold, at least three-fold, at least four fold, at least five-fold, at least 10 fold, at least 50 fold, at least 100 fold, at least 1000 fold, at least 10,000 fold, or at least 100,000 fold. In other aspects of this embodiment, a targeting domain disclosed herein has an equilibrium disassociation constant that is greater for its cognate target receptor relative to a non-cognate receptor by, *e.g*., about one-fold to about three-fold, about one-fold to about five-fold, about one-fold to about 10-fold, about one-fold to about 100-fold, about one-fold to about 1000-fold, about five-fold to about 10-fold, about five-fold to about 100-fold, about five-fold to about 1000-fold, about 10-fold to about 100-fold, about 10-fold to about 1000-fold, about 10-fold to about 10,000-fold, or about 10-fold to about 100,000-fold.

In another embodiment, a targeting domain disclosed herein may be one that preferentially interacts with a receptor located on a sensory neuron. In an aspect of this embodiment, the sensory neuron targeting domain is one whose cognate receptor is located exclusively on the plasma membrane of sensory neurons. In another aspect of this embodiment, the sensory neuron targeting domain is one whose cognate receptor is located primarily on the plasma membrane of sensory neuron. For example, a receptor for a sensory neuron targeting domain is located primarily on a sensory neuron when, *e.g.,* at least 60% of all cells that have a cognate receptor for a sensory neuron targeting domain on the surface of the plasma membrane are sensory neurons, at least 70% of all cells that have a cognate receptor for a sensory neuron targeting domain on the surface of the plasma membrane are sensory neurons, at least 80% of all cells that have a cognate receptor for a sensory neuron targeting domain on the surface of the plasma membrane are sensory neurons, or at least 90% of all cells that have a cognate receptor for a sensory neuron targeting domain on the surface of the plasma membrane are sensory neurons. In yet another aspect of this embodiment, the sensory neuron targeting domain is one whose cognate receptor is located on the plasma membrane of several types of cells, including sensory neurons. In still another aspect of this embodiment, the sensory neuron targeting domain is one whose cognate receptor is located on the plasma membrane of several types of cells, including sensory neurons, with the proviso that motor neurons are not one of the other types of cells.

In another embodiment, a targeting domain disclosed herein may be one that preferentially interacts with a receptor located on a sympathetic neuron. In an aspect of this embodiment, the sympathetic neuron targeting domain is one whose cognate receptor is located exclusively on the plasma membrane of sympathetic neurons. In another aspect of this embodiment, the sympathetic neuron targeting domain is one whose cognate receptor is located primarily on the plasma membrane of sympathetic neuron. For example, a receptor for a sympathetic neuron targeting domain is located primarily on a sympathetic neuron when, *e.g.,* at least 60% of all cells that have a cognate receptor for a sympathetic neuron targeting domain on the surface of the plasma membrane are sympathetic neurons, at least 70% of all cells that have a cognate receptor for a sympathetic neuron targeting domain on the surface of the plasma membrane are sympathetic neurons, at least 80% of all cells that have a cognate receptor for a sympathetic neuron targeting domain on the surface of the plasma membrane are sympathetic neurons, or at least 90% of all cells that have a cognate receptor for a sympathetic neuron targeting domain on the surface of the plasma membrane are sympathetic neurons. In yet another aspect of this embodiment, the sympathetic neuron targeting domain is one whose cognate receptor is located on the plasma membrane of several types of cells, including sympathetic neurons. In still another aspect of this embodiment, the sympathetic neuron targeting domain is one whose cognate receptor is located on the plasma membrane of several types of cells, including sympathetic neurons, with the proviso that motor neurons are not one of the other types of cells.

In another embodiment, a targeting domain disclosed herein may be one that preferentially interacts with a receptor located on a parasympathetic neuron. In an aspect of this embodiment, the parasympathetic neuron targeting domain is one whose cognate receptor is located exclusively on the plasma membrane of parasympathetic neurons. In another aspect of this embodiment, the parasympathetic neuron targeting domain is one whose cognate receptor is located primarily on the plasma membrane of parasympathetic neuron. For example, a receptor for a parasympathetic neuron targeting domain is located primarily on a parasympathetic neuron when, *e.g.,* at least 60% of all cells that have a cognate receptor for a parasympathetic neuron targeting domain on the surface of the plasma membrane are parasympathetic neurons, at least 70% of all cells that have a cognate receptor for a parasympathetic neuron targeting domain on the surface of the plasma membrane are parasympathetic neurons, at least 80% of all cells that have a cognate receptor for a parasympathetic neuron targeting domain on the surface of the plasma membrane are parasympathetic neurons, or at least 90% of all cells that have a cognate receptor for a parasympathetic neuron targeting domain on the surface of the plasma membrane are parasympathetic neurons. In yet another aspect of this embodiment, the parasympathetic neuron targeting domain is one whose cognate receptor is located on the plasma membrane of several types of cells, including parasympathetic neurons. In still another aspect of this embodiment, the parasympathetic neuron targeting domain is one whose cognate receptor is located on the plasma membrane of several types of cells, including parasympathetic neurons, with the proviso that motor neurons are not one of the other types of cells.

In another embodiment, a targeting domain disclosed herein is an opioid peptide targeting domain, a galanin peptide targeting domain, a PAR peptide targeting domain, a somatostatin peptide targeting domain, a neurotensin peptide targeting domain, a SLURP peptide targeting domain, an angiotensin peptide targeting domain, a tachykinin peptide targeting domain, a Neuropeptide Y related peptide targeting domain, a kinin peptide targeting domain, a melanocortin peptide targeting domain, or a granin peptide targeting domain, a glucagon like hormone peptide targeting domain, a secretin peptide targeting domain, a pituitary adenylate cyclase activating peptide (PACAP) peptide targeting domain, a growth hormone-releasing hormone (GHRH) peptide targeting domain, a vasoactive intestinal peptide (VIP) peptide targeting domain, a gastric inhibitory peptide (GIP) peptide targeting domain, a calcitonin peptide targeting domain, a visceral gut peptide targeting domain, a neurotrophin peptide targeting domain, a head activator (HA) peptide, a glial cell line-derived neurotrophic factor (GDNF) family of ligands (GFL) peptide targeting domain, a RF-amide related peptide (RFRP) peptide targeting domain, a neurohormone peptide targeting domain, or a neuroregulatory cytokine peptide targeting domain, an interleukin (IL) targeting domain, vascular endothelial growth factor (VEGF) targeting domain, an insulin-like growth factor (IGF) targeting domain, an epidermal growth factor (EGF) targeting domain, a Transformation Growth Factor-β (TGFβ) targeting domain, a Bone Morphogenetic Protein (BMP) targeting domain, a Growth and Differentiation Factor (GDF) targeting domain, an activin targeting domain, or a Fibroblast Growth Factor (FGF) targeting domain, or a Platelet-Derived Growth Factor (PDGF) targeting domain.

In an aspect of this embodiment, an opioid peptide targeting domain is an enkephalin peptide, a bovine adrenomedullary-22 (BAM22) peptide, an endomorphin peptide, an endorphin peptide, a dynorphin peptide, a nociceptin peptide, or a hemorphin peptide. In another aspect of this embodiment, an enkephalin peptide targeting domain is a Leu-enkephalin peptide, a Met-enkephalin peptide, a Met-enkephalin MRGL peptide, or a Met-enkephalin MRF peptide. In another aspect of this embodiment, a bovine adrenomedullary-22 peptide targeting domain is a BAM22 (1-12) peptide, a BAM22 (6-22) peptide, a BAM22 (8-22) peptide, or a BAM22 (1-22) peptide. In another aspect of this embodiment, an endomorphin peptide targeting domain is an endomorphin-1 peptide or an endomorphin-2 peptide. In another aspect of this embodiment, an endorphin peptide targeting domain an endorphin-α peptide, a neoendorphin-α peptide, an endorphin-β peptide, a neoendorphin-β peptide, or an endorphin-γ peptide. In another aspect of this embodiment, a dynorphin peptide targeting domain is a dynorphin A peptide, a dynorphin B (leumorphin) peptide, or a rimorphin peptide. In another aspect of this embodiment, a nociceptin peptide targeting domain is a nociceptin RK peptide, a nociceptin peptide, a neuropeptide 1 peptide, a neuropeptide 2 peptide, or a neuropeptide 3 peptide. In another aspect of this embodiment, a hemorphin peptide targeting domain is a LVVH7 peptide, a VVH7 peptide, a VH7 peptide, a H7 peptide, a LVVH6 peptide, a LVVH5 peptide, a VVH5 peptide, a LVVH4 peptide, or a LVVH3 peptide.

In an aspect of this embodiment, a galanin peptide targeting domain is a galanin peptide, a galanin message-associated peptide (GMAP) peptide, a galanin like protein (GALP) peptide, or an alarin peptide.

In an aspect of this embodiment, a PAR peptide targeting domain is a PAR1 peptide, a PAR2 peptide, a PAR3 peptide and a PAR4 peptide. In an aspect of this embodiment, a somatostatin peptide targeting domain is a somatostatin peptide or a cortistatin peptide. In an aspect of this embodiment, a neurotensin peptide targeting domain a neurotensin or a neuromedin N. In an aspect of this embodiment, a SLURP peptide targeting domain is a SLURP-1 peptide or a SLURP-2 peptide. In an aspect of this embodiment, an angiotensin peptide targeting domain is an angiotensin peptide.

In an aspect of this embodiment, a tachykinin peptide targeting domain is a Substance P peptide, a neuropeptide K peptide, a neuropeptide gamma peptide, a neurokinin A peptide, a neurokinin B peptide, a hemokinin peptide, or a endokinin peptide. In an aspect of this embodiment, a Neuropeptide Y related peptide targeting domain is a Neuropeptide Y peptide, a Peptide YY peptide, Pancreatic peptide peptide, a Pancreatic icosapeptide peptide, a Pancreatic Hormone domain peptide, a CXCL12 peptide, and a Sjogren syndrome antigen B peptide. In an aspect of this embodiment, a kinin peptide targeting domain is a bradykinin peptide, a kallidin peptide, a desArg9 bradykinin peptide, a desArg10 bradykinin peptide, a kininogen peptide, gonadotropin releasing hormone 1 peptide, chemokine peptide, an arginine vasopressin peptide.

In an aspect of this embodiment, a melanocortin peptide targeting domain comprises a melanocyte stimulating hormone peptide, an adrenocorticotropin peptide, a lipotropin peptide, or a melanocortin peptide derived neuropeptide. In an aspect of this embodiment, a melanocyte stimulating hormone peptide targeting domain comprises an α-melanocyte stimulating hormone peptide, a β-melanocyte stimulating hormone peptide, or a γ-melanocyte stimulating hormone peptide. In an aspect of this embodiment, an adrenocorticotropin peptide targeting domain comprises an adrenocorticotropin or a Corticotropin-like intermediary peptide. In an aspect of this embodiment, a lipotropin peptide targeting domain comprises a β-lipotropin peptide or a γ-lipotropin peptide.

In an aspect of this embodiment, a granin peptide targeting domain comprises a chromogranin A peptide, a chromogranin B peptide, a chromogranin C (secretogranin II) peptide, a secretogranin IV peptide, or a secretogranin VI peptide. In an aspect of this embodiment, a chromogranin A peptide targeting domain comprises a β-granin peptide, a vasostatin peptide, a chromostatin peptide, a pancreastatin peptide, a WE-14 peptide, a catestatin peptide, a parastatin peptide, or a GE-25 peptide. In an aspect of this embodiment, a chromogranin B peptide targeting domain comprises a GAWK peptide, an adrenomedullary peptide, or a secretolytin peptide. In an aspect of this embodiment, a chromogranin C peptide targeting domain comprises a secretoneurin peptide.

In an aspect of this embodiment, a glucagons-like hormone peptide targeting domain is a glucagon-like peptide -1, a glucagon-like peptide-2, a glicentin, a glicentin-related peptide (GRPP), a glucagon, or an oxyntomodulin (OXY). In an aspect of this embodiment, a secretin peptide targeting domain is a secretin peptide. In an aspect of this embodiment, a pituitary adenylate cyclase activating peptide targeting domain is a pituitary adenylate cyclase activating peptide. In an aspect of this embodiment, a growth hormone-releasing hormone peptide targeting domain a growth hormone-releasing hormone peptide. In an aspect of this embodiment, a vasoactive intestinal peptide targeting domain is a vasoactive intestinal peptide-1 peptide or a vasoactive intestinal peptide-2 peptide. In an aspect of this embodiment, a gastric inhibitory peptide targeting domain is a gastric inhibitory peptide. In an aspect of this embodiment, a calcitonin peptide targeting domain is a calcitonin peptide, an amylin peptide, a calcitonin-related peptide α, a calcitonin-related peptide β, and a islet amyloid peptide. In an aspect of this embodiment, a visceral gut peptide targeting domain is a gastrin peptide, a gastrin-releasing peptide, or a cholecystokinin peptide.

In an aspect of this embodiment, a neurotrophin peptide targeting domain is a nerve growth factor (NGF) peptide, a brain derived neurotrophic factor (BDNF) peptide, a neurotrophin-3 (NT-3) peptide, a neurotrophin-4/5 (NT-4/5) peptide, or an amyloid beta (A4) precursor protein neurotrophin (APP) peptide. In an aspect of this embodiment, a head activator peptide targeting domain is a head activator peptide. In an aspect of this embodiment, a glial cell line-derived neurotrophic factor family of ligands peptide targeting domain is a glial cell line-derived neurotrophic factor peptide, a Neurturin peptide, a Persephrin peptide, or an Artemin peptide. In an aspect of this embodiment, a RF-amide related peptide targeting domain a RF-amide related peptide-1, a RF-amide related peptide-2, a RF-amide related peptide-3, a neuropeptide AF, or a neuropeptide FF.

In an aspect of this embodiment, a neurohormone peptide targeting domain is a corticotropin-releasing hormone (CCRH), a parathyroid hormone (PTH), a parathyroid hormone-like hormone (PTHLH), a PHYH, a thyrotropin-releasing hormone (TRH), an urocortin-1 (UCN1), an urocortin-2 (UCN2), an urocortin-3 (UCN3), or an urotensin 2 (UTS2). In an aspect of this embodiment, a neuroregulatory cytokine peptide targeting domain is a ciliary neurotrophic factor peptide, a glycophorin-A peptide, a leukemia inhibitory factor peptide, a cardiotrophin-1 peptide, a cardiotrophin-like cytokine peptide, a neuroleukin peptide, and an onostatin M peptide. In an aspect of this embodiment, an IL peptide targeting domain is an IL-1 peptide, an IL-2 peptide, an IL-3 peptide, an IL-4 peptide, an IL-5 peptide, an IL-6 peptide, an IL-7 peptide, an IL-8 peptide, an IL-9 peptide, an IL-10 peptide, an IL-11 peptide, an IL-12 peptide, an IL-18 peptide, an IL-32 peptide, or an IL-33 peptide.

In an aspect of this embodiment, a VEGF peptide targeting domain is a VEGF-A peptide, a VEGF-B peptide, a VEGF-C peptide, a VEGF-D peptide, or a placenta growth factor (P1GF) peptide. In an aspect of this embodiment, an IGF peptide targeting domain is an IGF-1 peptide or an IGF-2 peptide. In an aspect of this embodiment, an EGF peptide targeting domain an EGF, a heparin-binding EGF-like growth factor (HB-EGF), a transforming growth factor-α (TGF-α), an amphiregulin (AR), an epiregulin (EPR), an epigen (EPG), a betacellulin (BTC), a neuregulin-1 (NRG1), a neuregulin-2 (NRG2), a neuregulin-3, (NRG3), or a neuregulin-4 (NRG4). In an aspect of this embodiment, a FGF peptide targeting domain is a FGF1 peptide, a FGF2 peptide, a FGF3 peptide, a FGF4 peptide, a FGF5 peptide, a FGF6 peptide, a FGF7 peptide, a FGF8 peptide, a FGF9 peptide, a FGF10 peptide, a FGF17 peptide, or a FGF18 peptide. In an aspect of this embodiment, a PDGF peptide targeting domain is a PDGFα peptide or a PDGFβ peptide.

In an aspect of this embodiment, a TGFβ peptide targeting domain is a TGFβ1 peptide, a TGFβ2 peptide, a TGFβ3 peptide, or a TGFβ4 peptide. In an aspect of this embodiment, a BMP peptide targeting domain is a BMP2 peptide, a BMP3 peptide, a BMP4 peptide, a BMP5 peptide, a BMP6 peptide, a BMP7 peptide, a BMP8 peptide, or a BMP10 peptide. In an aspect of this embodiment, a GDF peptide targeting domain is a GDF1 peptide, a GDF2 peptide, a GDF3 peptide, a GDF5 peptide, a GDF6 peptide, a GDF7 peptide, a GDF8 peptide, a GDF10 peptide, a GDF11 peptide, or a GDF15 peptide. In an aspect of this embodiment, an activin peptide targeting domain is an activin A peptide, an activin B peptide, an activin C peptide, an activin E peptide, or an inhibin A peptide.

As discussed above, naturally-occurring Clostridial toxins are organized into three functional domains comprising a linear amino-to-carboxyl single polypeptide order of the enzymatic domain (amino region position), the translocation domain (middle region position) and the binding domain (carboxyl region position). This naturally-occurring order can be referred to as the carboxyl presentation of the binding domain because the domain necessary for binding to the receptor is located at the carboxyl region position of the Clostridial toxin. However, it has been shown that Clostridial toxins can be modified by rearranging the linear amino-to-carboxyl single polypeptide order of the three major domains and locating a targeting moiety at the amino region position of a Clostridial toxin, referred to as amino presentation, as well as in the middle region position, referred to as central presentation.

Thus, a TEM can comprise a targeting domain in any and all locations with the proviso that TEM is capable of performing the intoxication process. Non-limiting examples include, locating a targeting domain at the amino terminus of a TEM; locating a targeting domain between a Clostridial toxin enzymatic domain and a Clostridial toxin translocation domain of a TEM; and locating a targeting domain at the carboxyl terminus of a TEM. Other non-limiting examples include, locating a targeting domain between a Clostridial toxin enzymatic domain and a Clostridial toxin translocation domain of a TEM. The enzymatic domain of naturally-occurring Clostridial toxins contains the native start methionine. Thus, in domain organizations where the enzymatic domain is not in the amino-terminal location an amino acid sequence comprising the start methionine should be placed in front of the amino-terminal domain. Likewise, where a targeting domain is in the amino-terminal position, an amino acid sequence comprising a start methionine and a protease cleavage site may be operably-linked in situations in which a targeting domain requires a free amino terminus, see, *e.g.,* Shengwen Li et al., *Degradable Clostridial Toxins,* U.S. Patent Application 11/572,512 (Jan. 23, 2007). In addition, it is known in the art that when adding a polypeptide that is operably-linked to the amino terminus of another polypeptide comprising the start methionine that the original methionine residue can be deleted.

A TEM disclosed herein may optionally comprise an exogenous protease cleavage site that allows the use of an exogenous protease to convert the single-chain polypeptide form of a TEM into its more active di-chain form. As used herein, the term "exogenous protease cleavage site" is synonymous with a "non-naturally occurring protease cleavage site" or "non-native protease cleavage site" and means a protease cleavage site that is not naturally found in a di-chain loop region from a naturally occurring Clostridial toxin.

Naturally-occurring Clostridial toxins are each translated as a single-chain polypeptide of approximately 150 kDa that is subsequently cleaved by proteolytic scission within a disulfide loop by a naturally-occurring protease. This cleavage occurs within the discrete di-chain loop region located between two cysteine residues that form a disulfide bridge and comprising an endogenous protease cleavage site. As used herein, the term "endogenous di-chain loop protease cleavage site" is synonymous with a "naturally occurring di-chain loop protease cleavage site" and refers to a naturally occurring protease cleavage site found within the di-chain loop region of a naturally occurring Clostridial toxin. This posttranslational processing yields a di-chain molecule comprising an approximately 50 kDa light chain, comprising the enzymatic domain, and an approximately 100 kDa heavy chain, comprising the translocation and cell binding domains, the light chain and heavy chain being held together by the single disulfide bond and non-covalent interactions. Recombinantly-produced Clostridial toxins generally substitute the naturally-occurring di-chain loop protease cleavage site with an exogenous protease cleavage site to facilitate production of a recombinant di-chain molecule. *See e.g.,* Dolly, J.O. et al., *Activatable Clostridial Toxins,* U.S. Patent No. 7,419,676 (Sep. 2, 2008).

Although TEMs vary in their overall molecular weight because the size of the targeting domain, the activation process and its reliance on an exogenous cleavage site is essentially the same as that for recombinantly-produced Clostridial toxins. *See e.g.,* Steward, et al., *Activatable Clostridial Toxins,* US 2009/0081730; Steward, et al., *Modified Clostridial Toxins with Enhanced Translocation Capabilities and Altered Targeting Activity For Non-Clostridial Toxin Target Cells,* U.S. Patent Application No. 11/776,075; Steward, et al., *Modified Clostridial Toxins with Enhanced Translocation Capabilities and Altered Targeting Activity for Clostridial Toxin Target Cells,* US 2008/0241881. In general, the activation process that converts the single-chain polypeptide into its di-chain form using exogenous proteases can be used to process TEMs having a targeting domain organized in an amino presentation, central presentation, or carboxyl presentation arrangement. This is because for most targeting domains the amino-terminus of the moiety does not participate in receptor binding. As such, a wide range of protease cleavage sites can be used to produce an active di-chain form of a TEM. However, targeting domains requiring a free amino-terminus for receptor binding require a protease cleavage site whose scissile bond is located at the carboxyl terminus. The use of protease cleavage site is the design of a TEM are described in, *e.g*., Steward, et al., Activatable Clostridial toxins, US 2009/0069238; Ghanshani, et al., Modified Clostridial Toxins Comprising an Integrated Protease Cleavage Site-Binding Domain, US 2011/0189162; and Ghanshani, et al., Methods of Intracellular Conversion of Single-Chain Proteins into their Di-chain Form, International Patent Application Serial No. PCT/US2011/22272.

Non-limiting examples of exogenous protease cleavage sites include, e.g., a plant papain cleavage site, an insect papain cleavage site, a crustacian papain cleavage site, an enterokinase protease cleavage site, a Tobacco Etch Virus protease cleavage site, a Tobacco Vein Mottling Virus protease cleavage site, a human rhinovirus 3C protease cleavage site, a human enterovirus 3C protease cleavage site, a subtilisin cleavage site, a hydroxylamine cleavage site, a SUMO/ULP-1 protease cleavage site, and a Caspase 3 cleavage site.

Thus, in an embodiment, a TEM can comprise an amino to carboxyl single polypeptide linear order comprising a targeting domain, a translocation domain, an exogenous protease cleavage site and an enzymatic domain. In an aspect of this embodiment, a TEM can comprise an amino to carboxyl single polypeptide linear order comprising a targeting domain, a Clostridial toxin translocation domain, an exogenous protease cleavage site and a Clostridial toxin enzymatic domain.

In another embodiment, a TEM can comprise an amino to carboxyl single polypeptide linear order comprising a targeting domain, an enzymatic domain, an exogenous protease cleavage site, and a translocation domai. In an aspect of this embodiment, a TEM can comprise an amino to carboxyl single polypeptide linear order comprising a targeting domain, a Clostridial toxin enzymatic domain, an exogenous protease cleavage site, a Clostridial toxin translocation domain.

In yet another embodiment, a TEM can comprise an amino to carboxyl single polypeptide linear order comprising an enzymatic domain, an exogenous protease cleavage site, a targeting domain, and a translocation domain. In an aspect of this embodiment, a TEM can comprise an amino to carboxyl single polypeptide linear order comprising a Clostridial toxin enzymatic domain, an exogenous protease cleavage site, a targeting domain, and a Clostridial toxin translocation domain.

In yet another embodiment, a TEM can comprise an amino to carboxyl single polypeptide linear order comprising a translocation domain, an exogenous protease cleavage site, a targeting domain, and an enzymatic domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

In another embodiment, a TEM can comprise an amino to carboxyl single polypeptide linear order comprising an enzymatic domain, a targeting domain, an exogenous protease cleavage site, and a translocation domain. In an aspect of this embodiment, a TEM can comprise an amino to carboxyl single polypeptide linear order comprising a Clostridial toxin enzymatic domain, a targeting domain, an exogenous protease cleavage site, a Clostridial toxin translocation domain.

In yet another embodiment, a TEM can comprise an amino to carboxyl single polypeptide linear order comprising a translocation domain, a targeting domain, an exogenous protease cleavage site and an enzymatic domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

In still another embodiment, a TEM can comprise an amino to carboxyl single polypeptide linear order comprising an enzymatic domain, an exogenous protease cleavage site, a translocation domain, and a targeting domain. In an aspect of this embodiment, a TEM can comprise an amino to carboxyl single polypeptide linear order comprising a Clostridial toxin enzymatic domain, an exogenous protease cleavage site, a Clostridial toxin translocation domain, and a targeting domain.

In still another embodiment, a TEM can comprise an amino to carboxyl single polypeptide linear order comprising a translocation domain, an exogenous protease cleavage site, an enzymatic domain and a targeting domain. In an aspect of this embodiment, a TEM can comprise an amino to carboxyl single polypeptide linear order comprising a Clostridial toxin translocation domain, a targeting domain, an exogenous protease cleavage site and a Clostridial toxin enzymatic domain.

Non-limiting examples of TEMs disclosed herein, including TEMs comprising a Clostridal toxin enzymatic domain, a Clostridial toxin translocation domain and a targeting domain, the use of an exogenous protease cleavage site, and the design of amino presentation, central presentation and carboxyl presentation TEMs are described in, *e.g.,* US 7,959,933, Activatable Recombinant Neurotoxins, US 7,897,157, Activatable Clostridial Toxins; US 7,833,535, Clostridial Toxin Derivatives and Methods for Treating Pain; US 7,811,584, Multivalent Clostridial Toxins; US 7,780,968, Clostridial Toxin Derivatives and Methods for Treating Pain; US 7,749,514, Activatable Clostridial Toxins, US 7,740,868, Activatable Clostridial Toxins; US 7,736,659, Clostridial Toxin Derivatives and Methods for Treating Pain; US 7,709,228, Activatable Recombinant Neurotoxins; US 7,704,512, Clostridial Toxin Derivatives and Methods for Treating Pain; US 7,659,092, Fusion Proteins; US 7,658,933, Non-Cytotoxic Protein Conjugates; US 7,622,127, Clostridial Toxin Derivatives and Methods for Treating Pain; US 7,514,088, Multivalent Clostridial Toxin Derivatives and Methods of Their Use; US 7,425,338, Clostridial Toxin Derivatives and Methods for Treating Pain; US 7,422,877, Activatable Recombinant Neurotoxins; US 7,419,676, Activatable Recombinant Neurotoxins; US 7,413,742, Clostridial Toxin Derivatives and Methods for Treating Pain; US 7,262,291, Clostridial Toxin Derivatives and Methods for Treating Pain; US 7,244,437, Clostridial Toxin Derivatives and Methods for Treating Pain; US 7,244,436, Clostridial Toxin Derivatives and Methods for Treating Pain; US 7,138,127, Clostridial Toxin Derivatives and Methods for Treating Pain; US 7,132,259, Activatable Recombinant Neurotoxins; US 7,056,729, Botulinum Neurotoxin-Substance P Conjugate or Fusion Protein for Treating Pain; US 6,641,820, Clostridial Toxin Derivatives and Methods to Treat Pain; US 6,500,436, Clostridial Toxin Derivatives and Methods for Treating Pain; US 2011/0091437, Fusion Proteins; US 2011/0070621, Multivalent Clostridial Toxins; US 2011/0027256, Fusion Proteins; US 2010/0247509, Fusion Proteins; US 2010/0041098, Modified Clostridial Toxins with Altered Targeting Capabilities for Clostridial Toxin Target Cells; US 2010/0034802, Treatment of Pain; US 2009/0162341, Non-Cytotoxic Protein Conjugates; US 2009/0087458, Activatable Recombinant Neurotoxins; US 2009/0081730, Activatable Recombinant Neurotoxins; US 2009/0069238, Activatable Clostridial Toxins; US 2009/0042270, Activatable Recombinant Neurotoxins; US 2009/0030182, Activatable Recombinant Neurotoxins; US 2009/0018081, Activatable Clostridial Toxins; US 2009/0005313, Activatable Clostridial Toxins; US 2009/0004224, Activatable Clostridial Toxins; US 2008/0317783, Clostridial Toxin Derivatives and Methods for Treating Pain; US 2008/0241881, Modified Clostridial Toxins with Enhanced Translocation Capabilities and Altered Targeting Activity for Clostridial Toxin Target Cells; WO 2006/099590, Modified Clostridial Toxins with Altered Targeting Capabilities for Clostridial Toxin Target Cells; WO 2006/101809, Modified Clostridial Toxins with Enhanced Targeting Capabilities for Endogenous Clostridial Toxin Receptor Systems; WO 2007/106115, Modified Clostridial Toxins with Altered Targeting Capabilities for Clostridial Toxin Target Cells; WO 2008/008803, Modified Clostridial Toxins with Enhanced Translocation Capabilities and Altered Targeting Activity for Clostridial Toxin Target Cells; WO 2008/008805, Modified Clostridial Toxins with Enhanced Translocation Capabilities and Altered Targeting Activity For Non-Clostridial Toxin Target Cells; WO 2008/105901, Modified Clostridial Toxins with Enhanced Translocation Capability and Enhanced Targeting Activity; WO 2011/020052, Methods of Treating Cancer Using Opioid Retargeted Endpeptidases; WO 2011/020056, Methods of Treating Cancer Using Galanin Retargeted Endpeptidases; WO 2011/020114, Methods of Treating Cancer Using Tachykinin Retargeted Endopeptidases; WO 2011/020115, Methods of Treating Cancer Using Growth Factor Retargeted Endopeptidases; WO 2011/020117, Methods of Treating Cancer Using Neurotrophin Retargeted Endopeptidases; WO 2011/020119, Methods of Treating Cancer Using Glucagon-Like Hormone Retargeted Endopeptidases.

Aspects of the present specification disclose, in part, a composition comprising a Clostridial toxin and a TEM as disclosed herein. A composition disclosed herein is generally administered as a pharmaceutical acceptable composition. As used herein, the term "pharmaceutically acceptable" means any molecular entity or composition that does not produce an adverse, allergic or other untoward or unwanted reaction when administered to an individual. As used herein, the term "pharmaceutically acceptable composition" is synonymous with "pharmaceutical composition" and means a therapeutically effective concentration of an active ingredient, such as, *e.g.,* any of the Clostridial toxins and TEMs disclosed herein. A pharmaceutical composition disclosed herein is useful for medical and veterinary applications. A pharmaceutical composition may be administered to an individual alone, or in combination with other supplementary active ingredients, agents, drugs or hormones. The pharmaceutical compositions may be manufactured using any of a variety of processes, including, without limitation, conventional mixing, dissolving, granulating, dragee-making, levigating, emulsifying, encapsulating, entrapping, and lyophilizing. The pharmaceutical composition can take any of a variety of forms including, without limitation, a sterile solution, suspension, emulsion, lyophilizate, tablet, pill, pellet, capsule, powder, syrup, elixir or any other dosage form suitable for administration.

Aspects of the present specification provide, in part, a composition comprising a Clostridial toxin and a TEM. It is envisioned that any of the compositions disclosed herein can be useful in a method of treating disclosed herein, with the proviso that the composition prevents or reduces a symptom associated with condition being treated. A Clostridial toxin and a TEM as disclosed herein may be provided as separate compositions or as part of a single composition. It is also understood that the two or more different Clostridial toxins and/or TEMs can be provided as separate compositions or as part of a single composition.

A pharmaceutical composition comprising a Clostridial toxin and a TEM may optionally include a pharmaceutically acceptable carrier that facilitates processing of an active ingredient into pharmaceutically acceptable compositions. As used herein, the term "pharmacologically acceptable carrier" is synonymous with "pharmacological carrier" and means any carrier that has substantially no long term or permanent detrimental effect when administered and encompasses terms such as "pharmacologically acceptable vehicle, stabilizer, diluent, additive, auxiliary or excipient." Such a carrier generally is mixed with an active compound, or permitted to dilute or enclose the active compound and can be a solid, semi-solid, or liquid agent. It is understood that the active ingredients can be soluble or can be delivered as a suspension in the desired carrier or diluent. Any of a variety of pharmaceutically acceptable carriers can be used including, without limitation, aqueous media such as, *e.g*., water, saline, glycine, hyaluronic acid and the like; solid carriers such as, *e.g*., mannitol, lactose, starch, magnesium stearate, sodium saccharin, talcum, cellulose, glucose, sucrose, magnesium carbonate, and the like; solvents; dispersion media; coatings; antibacterial and antifungal agents; isotonic and absorption delaying agents; or any other inactive ingredient. Selection of a pharmacologically acceptable carrier can depend on the mode of administration. Except insofar as any pharmacologically acceptable carrier is incompatible with the active ingredient, its use in pharmaceutically acceptable compositions is contemplated. Non-limiting examples of specific uses of such pharmaceutical carriers can be found in PHARMACEUTICAL DOSAGE FORMS AND DRUG DELIVERY SYSTEMS (Howard C. Ansel et al., eds., Lippincott Williams & Wilkins Publishers, 7th ed. 1999); REMINGTON: THE SCIENCE AND PRACTICE OF PHARMACY (Alfonso R. Gennaro ed., Lippincott, Williams & Wilkins, 20th ed. 2000); GOODMAN & GILMAN'S THE PHARMACOLOGICAL BASIS OF THERAPEUTICS (Joel G. Hardman et al., eds., McGraw-Hill Professional, 10th ed. 2001); and HANDBOOK OF PHARMACEUTICAL EXCIPIENTS (Raymond C. Rowe et al., APhA Publications, 4th edition 2003). These protocols are routine procedures and any modifications are well within the scope of one skilled in the art and from the teaching herein.

A pharmaceutical composition disclosed herein can optionally include, without limitation, other pharmaceutically acceptable components (or pharmaceutical components), including, without limitation, buffers, preservatives, tonicity adjusters, salts, antioxidants, osmolality adjusting agents, physiological substances, pharmacological substances, bulking agents, emulsifying agents, wetting agents, sweetening or flavoring agents, and the like. Various buffers and means for adjusting pH can be used to prepare a pharmaceutical composition disclosed herein, provided that the resulting preparation is pharmaceutically acceptable. Such buffers include, without limitation, acetate buffers, citrate buffers, phosphate buffers, neutral buffered saline, phosphate buffered saline and borate buffers. It is understood that acids or bases can be used to adjust the pH of a composition as needed. Pharmaceutically acceptable antioxidants include, without limitation, sodium metabisulfite, sodium thiosulfate, acetylcysteine, butylated hydroxyanisole and butylated hydroxytoluene. Useful preservatives include, without limitation, benzalkonium chloride, chlorobutanol, thimerosal, phenylmercuric acetate, phenylmercuric nitrate, a stabilized oxy chloro composition and chelants, such as, *e.g.,* DTPA or DTPA-bisamide, calcium DTPA, and CaNaDTPA-bisamide. Tonicity adjustors useful in a pharmaceutical composition include, without limitation, salts such as, *e.g*., sodium chloride, potassium chloride, mannitol or glycerin and other pharmaceutically acceptable tonicity adjustor. The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. It is understood that these and other substances known in the art of pharmacology can be included in a pharmaceutical composition. Exemplary pharmaceutical composition comprising a Clostridial toxin and a TEM are described in Hunt, et al., Animal Protein-Free Pharmaceutical Compositions, US Serial No. 12/331,816; and Dasari, et al., Clostridial Toxin Pharmaceutical Compositions, WO/2010/090677.

In an embodiment, a composition is a pharmaceutical composition comprising a TEM. In aspects of this embodiment, a pharmaceutical composition comprising a TEM further comprises a pharmacological carrier, a pharmaceutical component, or both a pharmacological carrier and a pharmaceutical component. In other aspects of this embodiment, a pharmaceutical composition comprising a TEM further comprises at least one pharmacological carrier, at least one pharmaceutical component, or at least one pharmacological carrier and at least one pharmaceutical component.

In another embodiment, a composition is a pharmaceutical composition comprising a Clostridial toxin. In aspects of this embodiment, a pharmaceutical composition comprising a Clostridial toxin further comprises a pharmacological carrier, a pharmaceutical component, or both a pharmacological carrier and a pharmaceutical component. In other aspects of this embodiment, a pharmaceutical composition comprising a Clostridial toxin further comprises at least one pharmacological carrier, at least one pharmaceutical component, or at least one pharmacological carrier and at least one pharmaceutical component.

In yet another embodiment, a composition is a pharmaceutical composition comprising a Clostridial toxin and a TEM. In aspects of this embodiment, a pharmaceutical composition comprising a Clostridial toxin and a TEM further comprises a pharmacological carrier, a pharmaceutical component, or both a pharmacological carrier and a pharmaceutical component. In other aspects of this embodiment, a pharmaceutical composition comprising a Clostridial toxin and a TEM further comprises at least one pharmacological carrier, at least one pharmaceutical component, or at least one pharmacological carrier and at least one pharmaceutical component.

The present invention encompasses botulinum toxin for use in a method for prophylactic treatment of Herpes virus recurrence by local administration of a botulinum neurotoxin to a mammal, such as a human patient. In certain embodiments the botulinum neurotoxin is a botulinum neurotoxin type A. The botulinum neurotoxin can be administered in an amount between about 1 unit and about 10,000 units and Herpes virus recurrence symptoms can be alleviated for between about 2 weeks and about 6 months. In particular examples, Herpes virus recurrence symptoms can be alleviated from about 2 months to about 6 months, or from about 4 to about 6 months, for example. In one aspect, the local administration step is carried out by direct administration of the Clostridial toxin, such as a botulinum neurotoxin, to at least one location of the peripheral nervous system of the patient.

In another embodiment, botulinum toxin for use in a method for prophylactic treatment of Herpes virus recurrence in a patient in need thereof is provided, where the method comprises a step of locally administering, by injection, said botulinum neurotoxin to the area near or at the infection site of the patient. In particular embodiments, the botulinum neurotoxin is injected into at least two locations, and in some examples at least three locations. In certain embodiments, botulinum neurotoxin is locally administered at or near the latency site of the Herpes virus. Exemplary amounts being from about 1 to about 2500 units of a botulinum neurotoxin type A, or any amount therebetween. When utilizing a botulinum neurotoxin type B for example, the administered amount can be from between about 1 unit and about 25,000 units, or from about 100 units to about 20,000 units or from about 500 units to about 15,000 units or any amount therebetween.

In particular embodiments, additional administration of botulinum neurotoxin to the infection site of the patient can be performed, for example from at least about 2 months to about 3 months or more after an initial administration of botulinum neurotoxin.

In particular embodiments, local administration of the botulinum neurotoxin type A is from about 1 unit to about 500 units, per injection site, per patient visit. In certain embodiments the local administration of botulinum neurotoxin type A for prophylactic treatment of Herpes virus recurrence is at a dose of about 1 unit, about 2 units, about 3 units, about 4 units, about 5 units, about 6.25 units, about 10 units, about 12.5 units, about 15 units, about 20 units, about 25 units, about 30 units, about 35 units, about 37.5 units, about 40 units, about 50 units, about 55 units, about 60 units, about 65 units, about 70 units, about 75 units, about 80 units, about 85 units, about 90 units, about 95 units, about 100 units, about 105 units, about 110 units, about 115 units, about 120 units, about 125 units, about 130 units, about 135 units, about 140 units, about 145 units, about 150 units, about 155 units, about 160 units, about 165 units, about 170 units, about 175 units, about 180 units, about 185 units, about 190 units, about 195 units, or about 200 units, per injection site, per patient visit.

In particular embodiments, local administration of a botulinum neurotoxin can be to two bilateral locations of the face or genital region. Additionally, administration of botulinum neurotoxin can be via transdermal, intramuscular, subcutaneous, subdermal, intradermal or implant administration, and can be to a trigeminal ganglia or sacral ganglia. In particular embodiments, the botulinum neurotoxin is administered by injection into the trigeminal nerve, facial nerve or pudendal nerve, and the botulinum neurotoxin is botulinum neurotoxin type A or type B.

In further embodiments, botulinum toxin for use in a method for prophylactic treatment of Herpes virus recurrence in a patient in need thereof is provided wherein the method comprises the step of locally administering a botulinum neurotoxin to the dorsal nerve, ilioinguinal nerve, genitofemoral nerve, pudendal nerve, external spermatic nerve, sacral nerve, sciatic nerve, perineal nerve or posterior scrotal nerve.

In certain embodiments, an appropriate needle for botulinum neurotoxin injection include needles of 30-guage or smaller, preferably from about 23-gauge to about 25-gauge, and the area is preferably cleaned, such as with alcohol, before injection. Local anesthetic cream, general anesthesia, sedation or any known be useful anesthetic may be utilized, and may be necessary, depending upon the particular patient (some patients being more sensitive than others) undergoing treatment in accordance with the present methods. In particular examples, topical use of an anesthetic cream, such as, for example benzocaine, butamben, dibucaine, lidocaine, oxybuprocaine, pramoxine, proparacaine, proxymetacaine, and tetracaine can be applied before administration of the botulinum neurotoxin via a needle.

In some instances, the dosage of botulinum neurotoxin administered can be increased until achieving the desired effect (e.g. until the patient no longer experiences a Herpes virus recurrence). In a particular embodiment, a first dosage can be from about 10 units to about 75 units of a botulinum neurotoxin, or from about 25 units to about 50 units of a botulinum neurotoxin, such as BOTOX®. If recurrence is observed, treatment dosage can be increased, as determined by the medical practitioner's evaluation of the particular case at hand, the dosage, for example, being increased up to about 100 or about 200 units of a botulinum neurotoxin. In such instances, the time between administration of increasing dosages of botulinum neurotoxin can be about 3 weeks, preferably about 1 month and most preferably about 2 months.

The present disclosure includes within its scope: (a) a botulinum neurotoxin complex as well as a pure botulinum neurotoxin obtained or processed by bacterial culturing, toxin extraction, concentration, preservation, freeze drying and/or reconstitution and; (b) modified or recombinant botulinum neurotoxin, that is botulinum neurotoxin that has had one or more amino acids or amino acid sequences deliberately deleted, modified or replaced by known chemical/biochemical amino acid modification procedures or by use of known host cell/recombinant vector recombinant technologies, as well as derivatives or fragments of botulinum neurotoxins so made, and includes botulinum neurotoxins with one or more attached non-native targeting moieties for a cell surface receptor present on a cell.

Preferably, because of its clinical history to successfully treat a number of indications, the present invention includes local administration of a botulinum type A or botulinum neurotoxin type B, although botulinum neurotoxin type B is used with a larger protein load, as compared to type A toxin. A botulinum neurotoxin type A used in the present invention can be a complex of toxin and non-toxin proteins, which together comprise a total molecular weight of up to about 900 kDa. Dosage ranges and amounts, like any pharmaceutical, are based upon size, age and health of the patient, as well as upon the particular commercial preparation of the botulinum neurotoxin used. As known in the art, therapeutic use of botulinum neurotoxins is tailored to the particular patient that is presented for treatment, e.g. prophylactic treatment of Herpes virus recurrence. A botulinum neurotoxin type B used in the present invention can be a pure toxin or complex of toxin and non-toxin proteins, which is used at a dose of between about 50 and about 20,000 units. Other botulinum neurotoxin serotypes may be used in proportion to the dosages and concentrations exemplified herein, according to their respective levels of biological activity. For example, most units listed in the instant disclosure are of BOTOX®, but different serotypes or strains of a botulinum neurotoxin may be used, and different amounts may be administered. For example, about 3-4 times of DYSPORT® (a botulinum neurotoxin type A complex available from Ipsen Inc.) than an amount of BOTOX® may be utilized; about 40-50 times of NEUROBLOC®/MYOBLOC® (a botulinum neurotoxin type B available from Solstice Neurosciences) than an amount of BOTOX® may be utilized; and about equivalent amounts, in units, of XEOMIN® (botulinum neurotoxin type A, by Merz Pharma) relative to BOTOX® units can be utilized, to achieve a desired therapeutic effect, respectively. The present invention also encompasses concurrent or serial administration of a mixture of two or more of the above neurotoxins to effectively treat a patient with Herpes virus infection.

In one embodiment, the amount of a Clostridial toxin and/or a TEM disclosed herein used will typically be an effective amount. As used herein, the term "effective amount" is synonymous with "therapeutically effective amount", "effective dose", or "therapeutically effective dose" and when used in reference to treating a multiple medical disorder refers to the minimum dose of a Clostridial toxin and a TEM necessary to achieve the desired therapeutic effect and includes a dose sufficient to reduce a symptom associated with a multiple medical disorder. An effective amount refers to the total amount of a Clostridial toxin and/or TEM administered to an individual in one setting. As such, an effective amount of a Clostridial toxin and/or TEM does not refer to the amount administered per site. For example, an effective amount of a Clostridial toxin administered to an individual may be 10U, whereas the amount of toxin administered per site may be 2U, *i.e.,* 2 U at five different sites. The effectiveness of a Clostridial toxin and a TEM disclosed herein in treating a multiple medical disorder can be determined by observing an improvement in an individual based upon one or more clinical symptoms, and/or physiological indicators associated with the condition. An improvement in a multiple medical disorder also can be indicated by a reduced need for a concurrent therapy.

In one embodiment, a combination therapy comprising a Clostridial toxin and a TEM comprises an effective amount of a Clostridial toxin is one where in combination with a TEM the amount of a Clostridial toxin achieves the desired therapeutic effect, but such an amount administered on its own would be ineffective. The appropriate effective amount of a Clostridial toxin and a TEM to be administered to an individual for a particular multiple medical disorder can be determined by a person of ordinary skill in the art by taking into account factors, including, without limitation, the type of multiple medical disorder, the location of the multiple medical disorder, the cause of the multiple medical disorder, the severity of the multiple medical disorder, the degree of relief desired, the duration of relief desired, the particular TEM and/or Clostridial toxin used, the rate of excretion of the particular TEM and/or Clostridial toxin used, the pharmacodynamics of the particular TEM and/or Clostridial toxin used, the nature of the other compounds to be included in the composition, the particular route of administration, the particular characteristics, history and risk factors of the individual, such as, *e.g.,* age, weight, general health and the like, or any combination thereof. Additionally, where repeated administration of a composition comprising a Clostridial toxin and/or a TEM is used, an effective amount of a Clostridial toxin and/or a TEM will further depend upon factors, including, without limitation, the frequency of administration, the half-life of the particular TEM and/or Clostridial toxin used, or any combination thereof. In is known by a person of ordinary skill in the art that an effective amount of a composition comprising a Clostridial toxin and/or TEM can be extrapolated from *in vitro* assays and *in vivo* administration studies using animal models prior to administration to humans.

Wide variations in the necessary effective amount are to be expected in view of the differing efficiencies of the various routes of administration. For instance, oral administration generally would be expected to require higher dosage levels than administration by intravenous or intravitreal injection. Similarly, systemic administration of a TEM would be expected to require higher dosage levels than a local administration. Variations in these dosage levels can be adjusted using standard empirical routines of optimization, which are well-known to a person of ordinary skill in the art. The precise therapeutically effective dosage levels and patterns are preferably determined by the attending physician in consideration of the above-identified factors. One skilled in the art will recognize that the condition of the individual can be monitored throughout the course of therapy and that the effective amount of a Clostridial toxin and a TEM disclosed herein that is administered can be adjusted accordingly.

In other aspects of this embodiment, a therapeutically effective amount of a TEM generally is in the range of about 1 fg to about 3.0 mg. In aspects of this embodiment, an effective amount of a TEM can be, *e.g*., about 100 fg to about 3.0 mg, about 100 pg to about 3.0 mg, about 100 ng to about 3.0 mg, or about 100 µg to about 3.0 mg. In other aspects of this embodiment, an effective amount of a TEM can be, *e.g*., about 100 fg to about 750 µg, about 100 pg to about 750 µg, about 100 ng to about 750 µg, or about 1 µg to about 750 µg. In yet other aspects of this embodiment, a therapeutically effective amount of a TEM can be, *e.g.,* at least 1 fg, at least 250 fg, at least 500 fg, at least 750 fg, at least 1 pg, at least 250 pg, at least 500 pg, at least 750 pg, at least 1 ng, at least 250 ng, at least 500 ng, at least 750 ng, at least 1 µg, at least 250 µg, at least 500 µg, at least 750 µg, or at least 1 mg. In still other aspects of this embodiment, a therapeutically effective amount of a composition comprising a TEM can be, *e.g.,* at most 1 fg, at most 250 fg, at most 500 fg, at most 750 fg, at most 1 pg, at most 250 pg, at most 500 pg, at most 750 pg, at most 1 ng, at most 250 ng, at most 500 ng, at most 750 ng, at most 1 µg, at least 250 µg, at most 500 µg, at most 750 µg, or at most 1 mg.

In yet other aspects of this embodiment, a therapeutically effective amount of a TEM generally is in the range of about 0.00001 mg/kg to about 3.0 mg/kg. In aspects of this embodiment, an effective amount of a TEM can be, *e.g*., about 0.0001 mg/kg to about 0.001 mg/kg, about 0.03 mg/kg to about 3.0 mg/kg, about 0.1 mg/kg to about 3.0 mg/kg, or about 0.3 mg/kg to about 3.0 mg/kg. In yet other aspects of this embodiment, a therapeutically effective amount of a TEM can be, *e.g.,* at least 0.00001 mg/kg, at least 0.0001 mg/kg, at least 0.001 mg/kg, at least 0.01 mg/kg, at least 0.1 mg/kg, or at least 1 mg/kg. In yet other aspects of this embodiment, a therapeutically effective amount of a TEM can be, *e.g.,* at most 0.00001 mg/kg, at most 0.0001 mg/kg, at most 0.001 mg/kg, at most 0.01 mg/kg, at most 0.1 mg/kg, or at most 1 mg/kg.

In aspects of this embodiment, a therapeutically effective amount of a composition comprising a Clostridial toxin reduces a symptom associated with a multiple medical disorder by, *e.g.,* at least 10%, at least 20%, at least 30%, at least 40%, at least 50%, at least 60%, at least 70%, at least 80%, at least 90% or at least 100%. In other aspects of this embodiment, a therapeutically effective amount of a composition comprising a Clostridial toxin reduces a symptom associated with a multiple medical disorder by, *e.g.,* at most 10%, at most 20%, at most 30%, at most 40%, at most 50%, at most 60%, at most 70%, at most 80%, at most 90% or at most 100%. In yet other aspects of this embodiment, a therapeutically effective amount of a composition comprising a Clostridial toxin reduces a symptom associated with a multiple medical disorder by, *e.g*., about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%. In still other aspects of this embodiment, a therapeutically effective amount of a Clostridial toxin is the dosage sufficient to inhibit neuronal activity for, *e.g.,* at least one week, at least one month, at least two months, at least three months, at least four months, at least five months, at least six months, at least seven months, at least eight months, at least nine months, at least ten months, at least eleven months, or at least twelve months.

In other aspects of this embodiment, a therapeutically effective amount of a Clostridial toxin generally is in the range of about 1 fg to about 3.0 µg. In other aspects of this embodiment, a therapeutically effective amount of a Clostridial toxin can be, *e.g.,* at least 1.0 pg, at least 10 pg, at least 100 pg, at least 1.0 ng, at least 10 ng, at least 100 ng, at least 1.0 µg, at least 10 µg, at least 100 µg, or at least 1.0 mg. In still other aspects of this embodiment, a therapeutically effective amount of a Clostridial toxin can be, *e.g.,* at most 1.0 pg, at most 10 pg, at most 100 pg, at most 1.0 ng, at most 10 ng, at most 100 ng, at most 1.0 µg, at most 10 µg, at most 100 µg, or at most 1.0 mg. In still other aspects of this embodiment, a therapeutically effective amount of a Clostridial toxin can be, *e.g*., about 1.0 pg to about 10 µg, about 10 pg to about 10 µg, about 100 pg to about 10 µg, about 1.0 ng to about 10 µg, about 10 ng to about 10 µg, or about 100 ng to about 10 µg. In still other aspects of this embodiment, a therapeutically effective amount of a Clostridial toxin can be from, *e.g*., about 1.0 pg to about 1.0 µg, about 10 pg to about 1.0 µg, about 100 pg to about 1.0 µg, about 1.0 ng to about 1.0 µg, about 10 ng to about 1.0 µg, or about 100 ng to about 1.0 µg. In other aspects of this embodiment, a therapeutically effective amount of a Clostridial toxin can be from, *e.g*., about 1.0 pg to about 100 ng, about 10 pg to about 100 ng, about 100 pg to about 100 ng, about 1.0 ng to about 100 ng, or about 10 ng to about 100 ng.

In yet other aspects of this embodiment, a therapeutically effective amount of a Clostridial toxin generally is in the range of about 0.1 U to about 2500 U. In other aspects of this embodiment, a therapeutically effective amount of a Clostridial toxin can be, *e.g.,* at least 1.0 U, at least 10 U, at least 100 U, at least 250 U, at least 500 U, at least 750 U, at least 1,000 U, at least 1,500 U, at least 2,000 U, or at least 2,500 U. In still other aspects of this embodiment, a therapeutically effective amount of a Clostridial toxin can be, *e.g.,* at most 1.0 U, at most 10 U, at most 100 U, at most 250 U, at most 500 U, at most 750 U, at most 1,000 U, at most 1,500 U, at most 2,000 U, or at most 2,500 U. In still other aspects of this embodiment, a therapeutically effective amount of a Clostridial toxin can be, *e.g*., about 1 U to about 2,000 U, about 10 U to about 2,000 U, about 50 U to about 2,000 U, about 100 U to about 2,000 U, about 500 U to about 2,000 U, about 1,000 U to about 2,000 U, about 1 U to about 1,000 U, about 10 U to about 1,000 U, about 50 U to about 1,000 U, about 100 U to about 1,000 U, about 500 U to about 1,000 U, about 1 U to about 500 U, about 10 U to about 500 U, about 50 U to about 500 U, about 100 U to about 500 U, about 1 U to about 100 U, about 10 U to about 100 U, about 50 U to about 100 U, about 0.1 U to about 1 U, about 0.1 U to about 5 U, about 0.1 U to about 10 U, about 0.1 U to about 15 U, about 0.1 U to about 20 U, about 0.1 U to about 25 U.

In still other aspects of this embodiment, a therapeutically effective amount of a Clostridial toxin generally is in the range of about 0.0001 U/kg to about 3,000 U/kg. In aspects of this embodiment, a therapeutically effective amount of a Clostridial toxin can be, *e.g.,* at least 0.001 U/kg, at least 0.01 U/kg, at least 0.1 U/kg, at least 1.0 U/kg, at least 10 U/kg, at least 100 U/kg, or at least 1000 U/kg. In other aspects of this embodiment, a therapeutically effective amount of a Clostridial toxin can be, *e.g.,* at most 0.001 U/kg, at most 0.01 U/kg, at most 0.1 U/kg, at most 1.0 U/kg, at most 10 U/kg, at most 100 U/kg, or at most 1000 U/kg. In yet other aspects of this embodiment, a therapeutically effective amount of a Clostridial toxin can be between, *e.g*., about 0.001 U/kg to about 1 U/kg, about 0.01 U/kg to about 1 U/kg, about 0.1 U/kg to about 1 U/kg, about 0.001 U/kg to about 10 U/kg, about 0.01 U/kg to about 10 U/kg, about 0.1 U/kg to about 10 U/kg about 1 U/kg to about 10 U/kg, about 0.001 U/kg to about 100 U/kg, about 0.01 U/kg to about 100 U/kg, about 0.1 U/kg to about 100 U/kg, about 1 U/kg to about 100 U/kg, or about 10 U/kg to about 100 U/kg. As used herein, the term "unit" or "U" is refers to the LD₅₀ dose, which is defined as the amount of a Clostridial toxin disclosed herein that killed 50% of the mice injected with the Clostridial toxin.

In aspects of this embodiment, a therapeutically effective amount of a combined therapy comprising a Clostridial toxin and a TEM reduces a symptom associated with a multiple medical disorder by, *e.g.,* at least 10%, at least 20%, at least 30%, at least 40%, at least 50%, at least 60%, at least 70%, at least 80%, at least 90% or at least 100%. In other aspects of this embodiment, a therapeutically effective amount of a combined therapy comprising a Clostridial toxin and a TEM reduces a symptom associated with a multiple medical disorder by, *e.g.,* at most 10%, at most 20%, at most 30%, at most 40%, at most 50%, at most 60%, at most 70%, at most 80%, at most 90% or at most 100%. In yet other aspects of this embodiment, a therapeutically effective amount of a combined therapy comprising a Clostridial toxin and a TEM reduces a symptom associated with a multiple medical disorder by, *e.g*., about 10% to about 100%, about 10% to about 90%, about 10% to about 80%, about 10% to about 70%, about 10% to about 60%, about 10% to about 50%, about 10% to about 40%, about 20% to about 100%, about 20% to about 90%, about 20% to about 80%, about 20% to about 20%, about 20% to about 60%, about 20% to about 50%, about 20% to about 40%, about 30% to about 100%, about 30% to about 90%, about 30% to about 80%, about 30% to about 70%, about 30% to about 60%, or about 30% to about 50%. In still other aspects of this embodiment, a therapeutically effective amount of a combined therapy comprising a Clostridial toxin and a TEM is the dosage sufficient to inhibit neuronal activity for, *e.g.,* at least one week, at least one month, at least two months, at least three months, at least four months, at least five months, at least six months, at least seven months, at least eight months, at least nine months, at least ten months, at least eleven months, or at least twelve months.

In other aspects of this embodiment, a therapeutically effective amount of a combined therapy comprising a Clostridial toxin and a TEM generally is in a Clostridial toxin: TEM molar ratio of about 1:1 to about 1:10,000. In other aspects of this embodiment, a therapeutically effective amount of a combined therapy comprising a Clostridial toxin and a TEM can be in a Clostridial toxin: TEM molar ratio of, *e.g.,* about 1:1, about 1:2, about 1:5, about 1:10, about 1:25, about 1:50, about 1:75, about 1:100, about 1:200, about 1:300, about 1:400, about 1:500, about 1:600, about 1:700, about 1:800, about 1:900, about 1:1000, about 1:2000, about 1:3000, about 1:4000, about 1:5000, about 1:6000, about 1:7000, about 1:8000, about 1:9000, or about 1:10,000. In yet other aspects of this embodiment, a therapeutically effective amount of combined therapy comprising a Clostridial toxin and a TEM can be in a Clostridial toxin: TEM molar ratio of, *e.g.,* at least 1:1, at least 1:2, at least 1:5, at least 1:10, at least 1:25, at least 1:50, at least 1:75, at least 1:100, at least 1:200, at least 1:300, at least 1:400, at least 1:500, at least 1:600, at least 1:700, at least 1:800, at least 1:900, at least 1:1000, at least 1:2000, at least 1:3000, at least 1:4000, at least 1:5000, at least 1:6000, at least 1:7000, at least 1:8000, at least 1:9000, or at least 1:10,000. In still other aspects of this embodiment, a therapeutically effective amount of a combined therapy comprising a Clostridial toxin and a TEM can be in a Clostridial toxin: TEM molar ratio of between, *e.g*., about 1:1 to about 1:10,000, about 1:10 to about 1:10,000, about 1:100 to about 1:10,000, about 1:500 to about 1:10,000, about 1:1000 to about 1:10,000, about 1:5000 to about 1:10,000, about 1:1 to about 1:1000, about 1:10 to about 1:1000, about 1:100 to about 1:1000, about 1:250 to about 1:1000, about 1:500 to about 1:1000, about 1:750 to about 1:1000, about 1:1 to about 1:500, about 1:10 to about 1:500, about 1:50 to about 1:500, about 1:100 to about 1:500, about 1:250 to about 1:500, about 1:1 to about 1:100, about 1:10 to about 1:100, about 1:25 to about 1:100, about 1:50 to about 1:100, or about 1:75 to about 1:100.

In yet other aspects of this embodiment, a therapeutically effective amount of a combined therapy comprising a Clostridial toxin and a TEM generally is in a range of about 0.01 U to about 50 U of Clostridial toxin and about 0.1 µg to about 1,000.0 µg of a TEM. In aspects of this embodiment, a therapeutically effective amount of a combined therapy comprising a Clostridial toxin and a TEM can be, *e.g*., about 0.1 U to about 10 U of a Clostridial toxin and about 10 µg to about 100 µg of a TEM, about 0.5 U to about 10 U of a Clostridial toxin and about 10 µg to about 100 µg of a TEM, about 1 U to about 10 U of a Clostridial toxin and about 100 µg to about 1,000 µg of a TEM.

Dosing can be single dosage or cumulative (serial dosing), and can be readily determined by one skilled in the art. For instance, treatment of a multiple medical disorder may comprise a one-time administration of an effective dose of a composition disclosed herein. As a non-limiting example, an effective dose of a composition disclosed herein can be administered once to an individual, *e.g*., as a single injection or deposition at or near the site exhibiting a symptom of a multiple medical disorder. Alternatively, treatment of a multiple medical disorder may comprise multiple administrations of an effective dose of a composition disclosed herein carried out over a range of time periods, such as, *e.g.,* daily, once every few days, weekly, monthly or yearly. As a non-limiting example, a composition disclosed herein can be administered once or twice yearly to an individual. The timing of administration can vary from individual to individual, depending upon such factors as the severity of an individual's symptoms. For example, an effective dose of a composition disclosed herein can be administered to an individual once a month for an indefinite period of time, or until the individual no longer requires therapy. A person of ordinary skill in the art will recognize that the condition of the individual can be monitored throughout the course of treatment and that the effective amount of a composition disclosed herein that is administered can be adjusted accordingly.

### Definitions

As used herein, the words or terms set forth below have the following definitions:

"About" or "approximately" as used herein means within an acceptable error range for the particular value as determined by one of ordinary skill in the art, which will depend in part on how the value is measured or determined, (*i.e.,* the limitations of the measurement system). For example, "about" can mean within 1 or more than 1 standard deviations, per practice in the art. Where particular values are described in the application and claims, unless otherwise stated, the term "about" means within an acceptable error range for the particular value.

"Active pharmaceutical ingredient" (API) means an ingredient that exerts an effect upon or after administration to a subject or patient. API's can include, for example, botulinum neurotoxins, and the like.

"Administration", or "to administer" means the step of giving *(i.e.* administering) a pharmaceutical composition to a subject, or alternatively a subject receiving a pharmaceutical composition. The pharmaceutical compositions disclosed herein can be locally administered by various methods. For example, intramuscular, intradermal, subcutaneous administration, intrathecal administration, intraperitoneal administration, topical (transdermal), instillation, and implantation (for example, of a slow-release device such as polymeric implant or miniosmotic pump) can all be appropriate routes of administration.

"Alleviating" means a reduction in the occurrence of a pain, of a headache, or of any symptom or cause of a condition or disorder. Thus, alleviating includes some reduction, significant reduction, near total reduction, and total reduction. "Biological activity" describes the beneficial or adverse effects of a drug on living matter. When a drug is a complex chemical mixture, this activity is exerted by the substance's active ingredient but can be modified by the other constituents. Biological activity can be assessed as potency or as toxicity by an *in vivo* LD₅₀ or ED₅₀ assay, or through an *in vitro* assay such as, for example, cell-based potency assays as described in U.S. Patent 8,198,034 and U.S. 20100233802.

"Botulinum neurotoxin" means a neurotoxin produced by Clostridium botulinum, as well as a botulinum neurotoxin (or the light chain or the heavy chain thereof) made recombinantly by a non-Clostridial species. The phrase "botulinum neurotoxin", as used herein, encompasses the botulinum neurotoxin serotypes A, B, C, D, E, F and G, and their subtypes and any other types of subtypes thereof, or any re-engineered proteins, analogs, derivatives, homologs, parts, sub-parts, in each case, of any of the foregoing. "Botulinum neurotoxin", as used herein, also encompasses a "modified botulinum neurotoxin". Further "botulinum neurotoxin" as used herein also encompasses a botulinum neurotoxin complex, (for example, the 300, 600 and 900kDa complexes), as well as the neurotoxic component of the botulinum neurotoxin (150 kDa) that is unassociated with the complex proteins.

"Clostridial neurotoxin" means a neurotoxin produced from, or native to, a Clostridial bacterium, such as Clostridium botulinum, Clostridium butyricum or Clostridium beratti, as well as a Clostridial neurotoxin made recombinantly by a non-Clostridial species.

"Entirely free (*i.e.* "consisting of" terminology) means that within the detection range of the instrument or process being used, the substance cannot be detected or its presence cannot be confirmed.

"Essentially free" (or "consisting essentially of") means that only trace amounts of the substance can be detected.

"Light chain" means the light chain of a clostridial neurotoxin. It has a molecular weight of about 50kDa, and can be referred to as the L chain, L, or as the proteolytic domain (amino acid sequence) of a botulinum neurotoxin.

"Heavy chain" means the heavy chain of a botulinum neurotoxin. It has a molecular weight of about 100kDa and can be referred to as the H chain, or as H.

H_{C} means a fragment (about 50kDa) derived from the H chain of a botulinum neurotoxin which is approximately equivalent to the carboxyl end segment of the H chain, or the portion corresponding to that fragment in the intact H chain. It is believed to be immunogenic and to contain the portion of the natural or wild type botulinum neurotoxin involved in high affinity, presynaptic binding to motor neurons.

H_{N} means a fragment (about 50kDa) derived from the H chain of a botulinum neurotoxin which is approximately equivalent to the amino end segment of the H chain, or the portion corresponding to that fragment in the intact in the H chain. It is believed to contain the portion of the natural or wild type botulinum neurotoxin involved in the translocation of the L chain across an intracellular endosomal membrane.

LH_{N} or L-H_{N} means a fragment derived from a clostridial neurotoxin that contains the L chain, or a functional fragment thereof coupled to the H_{N} domain It can be obtained from the intact clostridial neurotoxin by proteolysis, so as to remove or to modify the H_{C} domain.

"Local administration" means direct administration of a pharmaceutical at or to the vicinity of a site on or within an animal body, at which site a biological effect of the pharmaceutical is desired, such as via, for example, intramuscular injection, intra-dermal injection, subdermal injection, subcutaneous injection, placement of an implant for administration of the neurotoxin, or topical administration. Local administration excludes systemic routes of administration, such as intravenous or oral administration. Topical administration is a type of local administration in which a pharmaceutical agent is applied to a patient's skin.

"Modified botulinum neurotoxin" means a botulinum neurotoxin that has had at least one of its amino acids deleted, modified, or replaced, as compared to a native botulinum neurotoxin. Additionally, the modified botulinum neurotoxin can be a recombinantly produced neurotoxin, or a derivative or fragment of a recombinantly made neurotoxin. A modified botulinum neurotoxin retains the ability to bind to a botulinum neurotoxin receptor, be internalized into a nerve cell and enymatically cleave a SNARE protein. One example of a modified botulinum neurotoxin is a botulinum neurotoxin that has a light chain from one botulinum neurotoxin serotype (such as serotype E), and a heavy chain from a different botulinum neurotoxin serotype (such as serotype A).

"Mutation" means a structural modification of a naturally occurring protein or nucleic acid sequence. For example, in the case of nucleic acid mutations, a mutation can be a deletion, addition or substitution of one or more nucleotides in the DNA sequence. In the case of a protein sequence mutation, the mutation can be a deletion, addition or substitution of one or more amino acids in a protein sequence. For example, a specific amino acid comprising a protein sequence can be substituted for another amino acid, for example, an amino acid selected from a group which includes the amino acids alanine, aspargine, cysteine, aspartic acid, glutamic acid, phenylalanine, glycine, histidine, isoleucine, lysine, leucine, methionine, proline, glutamine, arginine, serine, threonine, valine, tryptophan, tyrosine or any other natural or non-naturally occurring amino acid or chemically modified amino acids. Mutations to a protein sequence can be the result of mutations to DNA sequences that when transcribed, and the resulting mRNA translated, produce the mutated protein sequence. Mutations to a protein sequence can also be created by fusing a peptide sequence containing the desired mutation to a desired protein sequence.

"Neurotoxin" includes Clostridial neurotoxins both as pure toxin and complexed with one to more non-toxin, toxin associated proteins, whether made by the native Clostridial bacterium or by recombinant means in a non-Clostridial species. "Botulinum neurotoxin" means non-complexed botulinum neurotoxin (i.e. pure botulinum neurotoxin molecule having a molecular weight of about 150 kDa) or as a complex (i.e. having a molecular weight of about 300 to about 900 kDa weight complex comprising a neurotoxin molecule and one or more associated non-toxic molecules).

"Patient" means a human subject receiving medical care from a physician.

"Peripherally administering" or "peripheral administration" means subdermal, intradermal, transdermal, or subcutaneous administration, but excludes intramuscular administration. "Peripheral" means in a subdermal location, and excludes visceral sites.

"Pharmaceutical composition" means a composition comprising an active pharmaceutical ingredient, such as, for example, a botulinum neurotoxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, in a lyophilized or vacuum dried condition, a solution formed after reconstitution of the lyophilized or vacuum dried pharmaceutical composition, or as a solution or solid which does not require reconstitution.

"Therapeutic formulation" means a formulation can be used for prophylactic treatment of Herpes recurrence.

"Therapeutically effective amount," as used herein, means an amount of a Clostridial neurotoxin, for example a botulinum neurotoxin type A, B, C, D, E, F and G, that ameliorates, or eliminates one or more symptoms of a particular disease or condition such as prophylactic treatment of Herpes recurrence.

"Topical administration" excludes systemic administration of the neurotoxin. In other words, and unlike conventional therapeutic transdermal methods, topical administration of botulinum neurotoxin does not result in significant amounts, such as the majority of, the neurotoxin passing into the circulatory system of the patient.

"Treating" means to alleviate (or to eliminate) at least one symptom, either temporarily or permanently. Here, this includes prevention of the development of a pustular lesion or ulcer. For example, the term "treating" can mean reducing a symptom of a condition characterized by a multiple medical disorder by, e.g., at least 20%, at least 30%, at least 40%, at least 50%, at least 60%, at least 70%, at least 80%, at least 90% or at least 100%.

"Variant" means a clostridial neurotoxin, such as wild-type botulinum neurotoxin serotype A, B, C, D, E, F or G, that has been modified by the replacement, modification, addition or deletion of at least one amino acid relative to wild-type botulinum neurotoxin, which is recognized by a target cell, internalized by the target cell, and catalytically cleaves a SNARE (SNAP (Soluble NSF Attachment Protein) Receptor) protein in the target cell. A variant neurotoxin component can comprise a variant light chain of a botulinum neurotoxin having one or more amino acids substituted, modified, deleted and/or added. This variant light chain may have the same or better ability to prevent exocytosis, for example, the release of neurotransmitter vesicles. Additionally, the biological effect of a variant may be decreased compared to the parent chemical entity. For example, a variant light chain of a botulinum neurotoxin type A having an amino acid sequence removed may have a shorter biological persistence than that of the parent (or native) botulinum neurotoxin type A light chain. A variant may also have a substituted binding domain, so that it binds to a different cell than a wild-type botulinum neurotoxin. A variant may also include toxin described in by Steward et al., *Degradable Clostridial Toxins,* US Patent 8,512,992 (August 20, 2013).

"Vehicle" or "reconstitution vehicle" means a liquid composition that can be used to reconstitute a solid botulinum formulation into a liquid botulinum pharmaceutical composition.

"Wild type neuronal binding moiety" means that portion of a neurotoxin which is native to the neurotoxin and which exhibits a specific binding affinity for a receptor on a neuron. Thus, wild type or native neuronal binding moiety excludes a binding moiety with is not native to the neurotoxin.

### Botulinum toxin for use in methods of treatment

In at least one embodiment, botulinum toxin for use in a method of prophylactic treatment of Herpes recurrence is provided wherein a therapeutic amount of said botulinum neurotoxin is injected locally: (i) proximally at the infection site; and (ii) additional administration into the nerve wherein a dormant Herpes virus is maintained or surrounding muscles.

In at least one embodiment, botulinum toxin for use in a method of prophylactic treatment of Herpes recurrence is provided wherein a therapeutic amount of said botulinum neurotoxin is injected locally into the nerve wherein a dormant Herpes virus is maintained or surrounding muscles.

In at least one embodiment, botulinum toxin for use in a method of prophylactic treatment of Herpes recurrence is provided wherein injection of a therapeutic amount of said botulinum neurotoxin is used to block nerve pathways through the pudendal nerve. In this method, the dormant Herpes virus does not reactivate and latency is maintained.

In another embodiment, botulinum toxin for use in a method of prophylactic treatment of Herpes recurrence is provided wherein injection of a therapeutic amount of botulinum neurotoxin is used to block nerve pathways through the ilioinguinal nerve, the genital branch of the genitofemoral nerve, the perineal branch of the femoral cutaneous nerve and the perineal nerve. In this method, the dormant Herpes virus does not reactivate and latency is maintained.

In exemplary embodiments, the area that is to receive the botulinum neurotoxin administration is first cleaned utilizing alcohol, such as by utilizing an alcohol wipe, for example. Local anesthetic (as disclosed herein) is then applied to the cleaned area. The anesthetic can be applied topically with local sterile single use local anesthetic (LA) gel or cream (eg, lidocaine gel, cream) and, when ready the injection sites can be sterilized.

### EXAMPLES

The following examples illustrate embodiments and aspects of the present invention and are not intended to limit the scope of the present invention.

### Example 1

In a study of annualized rates of prevalence of Herpes recurrence, Botox® treated subjects were compared to placebo (saline) treatment. The number of Herpes recurrences was slightly decreased for crow's feet line treatments, and more greatly decreased for glabellar line treatments and migraine treatments with Botox® relative to placebo.

### Example 2

In a prophetic example, all subjects would have a known history of Herpes. Subjects are expected to have a decreased reactivation following administration of a therapeuticly effective dose of a botulinum neurotoxin at injection sites targeting the nerves where the Herpes visus hides. Nerves for targeted injection include but are not limited to the trigeminal, lower cervical, thoracic, lumbar and sacral.

Alternatively, the subjects could have been administed a TEM instead of a botulinum neurotoxin.

In another alternative example, the subjects could have been administered a combination of TEM and neurotoxin.

### Example 3

In a prophetic example, a botulinum neurotoxin is administered to subjects with oral mucosa herpes infection. A dosage of up to 40 U Botox® could be administered (2.5 U per side in the upper and lower orbicularis oculi muscle areas, 2.5U per side in the upper and lower orbicularis oris muscle areas and 10 U on each side of the face in the submalar area). Alternatively, the same injection pattern but a therapeutically effective dose of Dysport®, Xeomin® or other neurotoxin could be administered.

Alternatively, the subjects could have been administed a TEM instead of a botulinum neurotoxin.

In another alternative example, the subjects could have been administered a combination of TEM and neurotoxin.

## Claims

1. Botulinum neurotoxin for use in a method for the prophylactic treatment of a Herpes recurrence in a patient, the method comprising the step of locally administering a therapeutically effective amount of said botulinum neurotoxin to a nerve or in the proximity of a nerve of the patient, thereby prophylactically treating the Herpes recurrence in a patient.

2. Botulinum neurotoxin for use of claim 1, wherein the nerve maintains a Herpes virus in the lytic stage.

3. Botulinum neurotoxin for use of claim 1, wherein the nerve is located within a trigeminal ganglion.

4. Botulinum neurotoxin for use of claim 1, wherein the nerve is located within a sacral ganglion.

5. Botulinum neurotoxin for use of claim 1, wherein the nerve is located at the site of infection.

6. Botulinum neurotoxin for use of claim 1, wherein the nerve is a trigeminal nerve.

7. Botulinum neurotoxin for use of claim 1, wherein the nerve is a facial nerve.

8. Botulinum neurotoxin for use of claim 1, wherein the nerve is a pudendal nerve.

9. Botulinum neurotoxin for use of claim 1, wherein the amount of botulinum neurotoxin is less than that which is needed to paralyze a muscle.

10. Botulinum neurotoxin for use of claim 1, wherein between about 2 and about 500 units of botulinum neurotoxin is administered.

11. Botulinum neurotoxin for use of claim 1, wherein the administration is by topical administration.

12. Botulinum neurotoxin for use of claim 1, wherein the administration is by subcutaneous administration.

13. Botulinum neurotoxin for use of claim 1, wherein administration is transdermal or subdermal or intradermal.

## Patentansprüche

1. Botulinum-Neurotoxin zur Verwendung in einem Verfahren zur prophylaktischen Behandlung eines Herpesrezidivs bei einem Patienten, wobei das Verfahren den Schritt der lokalen Verabreichung einer therapeutisch wirksamen Menge des Botulinum-Neurotoxins an einen Nerv oder in der Nähe eines Nervs des Patienten umfasst, wodurch das Herpesrezidiv bei einem Patienten prophylaktisch behandelt wird.

2. Botulinum-Neurotoxin zur Verwendung gemäss Anspruch 1, wobei der Nerv ein Herpesvirus im lytischen Stadium beibehält.

3. Botulinum-Neurotoxin zur Verwendung gemäss Anspruch 1, wobei sich der Nerv in einem Ganglion trigeminale befindet.

4. Botulinum-Neurotoxin zur Verwendung gemäss Anspruch 1, wobei sich der Nerv in einem Sakralganglion befindet.

5. Botulinum-Neurotoxin zur Verwendung gemäss Anspruch 1, wobei sich der Nerv an der Infektionsstelle befindet.

6. Botulinum-Neurotoxin zur Verwendung gemäss Anspruch 1, wobei der Nerv ein Trigeminus ist.

7. Botulinum-Neurotoxin zur Verwendung gemäss Anspruch 1, wobei der Nerv ein Gesichtsnerv ist.

8. Botulinum-Neurotoxin zur Verwendung gemäss Anspruch 1, wobei der Nerv ein Pudendusnerv ist.

9. Botulinum-Neurotoxin zur Verwendung gemäss Anspruch 1, wobei die Menge an Botulinum-Neurotoxin geringer ist als diejenige, die benötigt wird, um einen Muskel zu lähmen.

10. Botulinum-Neurotoxin zur Verwendung gemäss Anspruch 1, wobei zwischen etwa 2 und etwa 500 Einheiten Botulinum-Neurotoxin verabreicht werden.

11. Botulinum-Neurotoxin zur Verwendung gemäss Anspruch 1, wobei die Verabreichung durch topische Verabreichung erfolgt.

12. Botulinum-Neurotoxin zur Verwendung gemäss Anspruch 1, wobei die Verabreichung durch subkutane Verabreichung erfolgt.

13. Botulinum-Neurotoxin zur Verwendung gemäss Anspruch 1, wobei die Verabreichung transdermal oder subdermal oder intradermal erfolgt.

## Revendications

1. Neurotoxine botulique pour utilisation dans un procédé de traitement prophylactique d'une récurrence de l'herpès chez un patient, le procédé comprenant l'étape d'administration locale d'une quantité thérapeutiquement efficace de ladite neurotoxine botulique à un nerf ou à proximité d'un nerf du patient, traitant de la sorte au plan prophylactique la récurrence de l'herpès chez un patient.

2. Neurotoxine botulique pour utilisation selon la revendication 1, dans laquelle le nerf entretient un virus de l'herpès au stade lytique.

3. Neurotoxine botulique pour utilisation selon la revendication 1, dans laquelle le nerf est situé dans un ganglion trigéminal.

4. Neurotoxine botulique pour utilisation selon la revendication 1, dans laquelle le nerf est situé dans un ganglion sacré.

5. Neurotoxine botulique pour utilisation selon la revendication 1, dans laquelle le nerf est situé sur le site d'infection.

6. Neurotoxine botulique pour utilisation selon la revendication 1, dans laquelle le nerf est un nerf trigéminal.

7. Neurotoxine botulique pour utilisation selon la revendication 1, dans laquelle le nerf est un nerf facial.

8. Neurotoxine botulique pour utilisation selon la revendication 1, dans laquelle le nerf est un nerf pudendal.

9. Neurotoxine botulique pour utilisation selon la revendication 1, dans laquelle la quantité de neurotoxine botulique est inférieure à celle qui est nécessaire pour paralyser un muscle.

10. Neurotoxine botulique pour utilisation selon la revendication 1, dans laquelle entre environ 2 et environ 500 unités de neurotoxine botulique sont administrées.

11. Neurotoxine botulique pour utilisation selon la revendication 1, dans laquelle l'administration se fait par administration topique.

12. Neurotoxine botulique pour utilisation selon la revendication 1, dans laquelle l'administration se fait par administration sous-cutanée.

13. Neurotoxine botulique pour utilisation selon la revendication 1, dans laquelle l'administration est transdermique ou sous-dermique ou intradermique.
